(19) Europäisches Patentamt
European Patent Office
Office européen des brevets

(11) EP 3 347 125 B1

(12) EUROPÄISCHE PATENTSCHRIFT

(45) Veröffentlichungstag und Bekanntmachung des Hinweises auf die Patenterteilung:
**06.05.2020 Patentblatt 2020/19**

(21) Anmeldenummer: **16744299.5**

(22) Anmeldetag: **07.07.2016**

(51) Int Cl.:
***B01J 20/289*** (2006.01) ***B01J 20/32*** (2006.01)
***B01D 15/20*** (2006.01) ***B01D 15/36*** (2006.01)
***C07K 1/16*** (2006.01) ***C07K 1/18*** (2006.01)
***C12N 9/36*** (2006.01)

(86) Internationale Anmeldenummer:
**PCT/EP2016/001168**

(87) Internationale Veröffentlichungsnummer:
**WO 2017/041868 (16.03.2017 Gazette 2017/11)**

# (54) ADSORPTIONSMEDIUM, VERFAHREN ZU DESSEN HERSTELLUNG, SOWIE VERWENDUNG DESSELBEN ZUR AUFREINIGUNG VON BIOMOLEKÜLEN

ADSORPTION MEDIUM, METHOD FOR PRODUCTION THEREOF, AND USE THEREOF FOR PURIFICATION OF BIOMOLECULES

MILIEU D'ADSORPTION, PROCÉDÉ POUR LE PRÉPARER ET SON UTILISATION POUR PURIFIER DES BIOMOLÉCULES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.09.2015 DE 102015011884**

(43) Veröffentlichungstag der Anmeldung:
**18.07.2018 Patentblatt 2018/29**

(73) Patentinhaber: **Sartorius Stedim Biotech GmbH**
**37079 Göttingen (DE)**

(72) Erfinder:
- **VILLAIN, Louis**
  **30451 Hannover (DE)**
- **TAFT, Florian**
  **37085 Göttingen (DE)**
- **SCHWELLENBACH, Jan**
  **37127 Dransfeld (DE)**

(74) Vertreter: **Müller-Boré & Partner**
**Patentanwälte PartG mbB**
**Friedenheimer Brücke 21**
**80639 München (DE)**

(56) Entgegenhaltungen:
**EP-A1- 1 224 975 WO-A1-2014/067605**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die vorliegende Erfindung betrifft ein Adsorptionsmedium, insbesondere ein Chromatographiemedium, ein Verfahren zu dessen Herstellung, sowie die Verwendung des erfindungsgemäßen Adsorptionsmediums bzw. eines erfindungsgemäß hergestellten Adsorptionsmediums zur Aufreinigung von Biomolekülen.

Als "Adsorptionsmedium" werden Adsorbentien bezeichnet, die über funktionelle Oberflächengruppen, oftmals auch als "chromatographisch aktive Zentren" und/oder "Liganden" bezeichnet, verfügen, die mit bestimmten Komponenten von Fluiden selektiv Bindungen eingehen können. Zielsubstanz(en) und/oder Kontaminant(en) werden erfindungsgemäß als "Adsorbenden" bezeichnet, wobei es sich auch um mehrere unterschiedliche Substanzen handeln kann. Adsorbenden können Einzelmoleküle, Assoziate oder Partikel sein, bei denen es sich vorzugsweise um Proteine oder andere Substanzen biologischen Ursprungs handelt.

Beispielhaft können ais mit den bzw. dem Adsorbenden wechseiwirkende Liganden Ionenaustauscher, Chelatbildner und Schwermetallchelate, thiophile, hydrophobe Liganden verschiedener Kettenlängen und Konfigurationen, "Reversed Phase"-Systeme, Farbstoffliganden, Affinitätsliganden, Aminosäuren, Coenzyme, Cofaktoren und deren Analoga, Substrate und deren Analoga, endokrine und exokrine Substanzen, wie Hormone und hormonähnlich wirkende Stoffe, Effektoren und deren Analoga, Enzym-Substrate, Enzym-Inhibitoren und deren Analoga, Fettsäuren, Fettsäurederivate, konjugierte Fettsäuren und deren Analoga, Nukleinsäuren, wie DNA, RNA und deren Analoga und Derivate (einzel-, doppel- und/oder mehrsträngig), sowie Peptidnukleinsäuren und deren Derivate, Viren, Virenpartikel, Monomere und deren Analoga und Derivate, Oligo- bis Polymere und deren Analoga und Derivate, hochmolekulare Kohlenhydrate, die linear oder verzweigt, nichtsubstituiert oder substituiert sein können, polymere Glycokonjugate, wie Heparin, Amylose, Cellulose, Chitin, Chitosan und deren Mono- und Oligomere und Derivate und Analoga davon, Lignin und dessen Derivate und Analoga, andere biologischchemische Liganden, wie Oligo- und Polypeptide, z.B. Proteine und ihre Oligomere, Multimere, Untereinheiten sowie Teile davon, insbesondere Lectine, Antikörper, Fusionsproteine, Haptene, Enzyme und Untereinheiten sowie Teile davon, Strukturproteine, Rezeptoren und Effektoren sowie Teile davon, des weiteren Xenobiotika, Pharmazeutika und pharmazeutische Wirkstoffe, Alkaloide, Antibiotika, Biomimetika usw. genannt werden.

Ein Adsorbens kann gleichzeitig auch zwei oder mehrere Arten der funktionellen Gruppen auf seiner inneren und äußeren Oberfläche tragen.

Die Bindung der Adsorbenden an das Adsorbens kann reversibel oder irreversibel sein, in jedem Fall ermöglicht sie ihre Abtrennung von den Fluiden, bei denen es sich beispielsweise um wässrige Flüssigkeiten handelt und die im Folgenden "Medien" genannt werden. Unter dem Begriff "Elution" werden die Desorption und die damit einhergehenden Spülschritte etc. zusammengefasst, und das zur Elution verwendete Medium ist das "Eluens". Die Komponenten können eine oder mehrere Zielsubstanzen und/oder eine oder mehrere Kontaminanten darstellen. "Zielsubstanzen" sind Wertstoffe, die aus dem Medium in angereicherter oder reiner Form gewonnen werden sollen. Zielprodukte können beispielsweise rekombinante Proteine, wie z.B. monoklonale Antikörper sein. "Kontaminanten" sind Stoffe, deren Abwesenheit oder Entfernung aus dem Fluid aus technischen, regulatorischen oder sonstigen Gründen erforderlich oder wünschenswert ist. Kontaminanten können beispielsweise Viren, Proteine, Aminosäuren, Nukleinsäuren, Endotoxine, Pröteinaggregate, Liganden oder deren Teile sein. Für die Entfernung von Kontaminanten, die als "negative Adsorption" bezeichnet wird, kann (darf) die Adsorption irreversibel verlaufen, wenn das Adsorbens nur einmal verwendet werden soll. Bei der Adsorption der Zielsubstanz(en) muss der Vorgang reversibel verlaufen. Es kann entweder eine bloße Anreicherung oder eine Auftrennung in mehrere Zielsubstanzen durchgeführt werden, wobei im letzteren Fall entweder die Adsorption, die Desorption oder beide selektiv erfolgen können.

Den Vorgang bezeichnet man als adsorptive Stofftrennung oder Chromatographie. Herkömmliche Adsorbentien für die Chromatographie sind entweder partikulär geformt und werden in Form von Schüttungen in Säulen betrieben, oder liegen in Form von Adsorptionsmembranen vor, die sich meist in Modulen befinden, deren Bauformen den in der Membranfiltration üblichen entsprechen (z.B. Wickelmodul, Stapelmodul etc.). Allen Adsorbentien ist üblicherweise die Grundanforderung nach möglichst niedriger unspezifischer Adsorption gemeinsam.

Wie vorstehend aufgeführt, ist im Stand der Technik eine Vielzahl von synthetischen und natürlichen Liganden bekannt. Der Bindung des Liganden an den Träger kann eine "Aktivierung" des Trägers vorausgehen, also die Einführung von reaktiven, funktionellen Gruppen, die zur spontanen Bindung des Liganden befähigt sind. Seltener weist der Ligand selbst eine reaktive Gruppe auf, wie z.B. die als Farbstoffliganden dienenden Reaktivfarbstoffe aus der Textilindustrie. Methoden zur Bindung von funktionellen Gruppen sind dem Fachmann an sich bekannt (z.B. aus Greg T. Hermanson, A. Krishna Mallia, Paul K. Smith, Immobilized Affinity Ligand Techniques, Academic Press, INC, 1992).

Die Filtration, Aufreinigung oder Entfernung von Biomolekülen wie Proteinen, Aminosäuren, Nukleinsäuren, Viren oder Endotoxinen aus flüssigen Medien ist von großem Interesse für die biopharmazeutische Industrie. Die meisten Anwendungen der Kontaminantenentfernung werden zurzeit mit konventionellen Chromatographiegelen oder Chromatographiemembranen betrieben.

So beschreiben zum Beispiel WO 2014/171437 A1 und WO 2014/034644 A1 poröse Chromatographiemedien für die Ionenaustauschchromatographie, beispielsweise für die Aufreinigung von Antikörpern, wobei es sich bei den

Medien um Membranen, Fasern oder Perlen handeln kann. Sowohl WO 2014/171437 A1 als auch WO 2014/034644 A1 lehren dabei, auf poröse Oberflächen von Chromatographiematrizen durch Atomtransferradikalpolymerisation ("*atom transfer radical polymerization"* (ATRP)) Propfketten aus Copolymeren aufzubringen, wobei die Propfketten unmittelbar auf der porösen Oberfläche fixiert sind. Die Propfketten aus Copolymeren tragen dabei die chromatographisch aktiven Zentren (Liganden), die mit den Adsorbenden dann eine selektive Bindung eingehen können.

WO 2014/067605 A1 offenbart ein Chromatographiemedium, umfassend eine Chromatographiematrix aus kontrolliert porösem Glas (CPG), polymere Abstandshalterelemente aus Polyacrylaten, die an die Oberfläche des kontrolliert porösen Glas gebunden sind, und Polymerketten aus Protein A, wobei die Protein A-Polymerketten an die polymeren Abstandshalterelemente gebunden sind. Die im Stand der Technik bekannten Adsorptionsmedien zeigen jedoch oftmals für große Zielmoleküle eine geringe Zugänglichkeit zu den Polymerketten, welche die chromatographisch aktiven Zentren tragen. Bei einer Bindungskonkurrenz zwischen kleinen und großen Zielmolekülen werden daher kleinere Zielmoleküle ungewünscht bevorzugt gebunden. Zudem werden die chromatographisch aktiven Zentren (Liganden) oftmals nicht optimal als Bindungsstellen ausgenutzt, so dass ein hoher Bedarf an Liganden in der Polymerschicht besteht, was zu hohen Herstellungskosten führen kann.

Der Erfindung liegt daher die Aufgabe zugrunde, ein Adsorptionsmedium bereitzustellen, das für große Zielmoleküle eine erhöhte Zugänglichkeit zu den Polymerketten, welche die chromatographisch aktiven Zentren tragen, aufweist, das große und kleine Zielmoleküle in gleichem Maße bindet und in dem die chromatographisch aktiven Zentren (Liganden) optimal als Bindungsstellen ausgenutzt werden können, so dass die Anzahl der Liganden im Adsorptionsmedium reduziert werden kann.

Diese Aufgabe wird durch die in den Ansprüchen gekennzeichneten Ausführungsformen der vorliegenden Erfindung gelöst.

Erfindungsgemäß wird ein Adsorptionsmedium, insbesondere ein Chromatographiemedium, bereitgestellt, umfassend eine Chromatographiematrix; polymere Abstandshalterelemente, die an die Oberfläche der Chromatographiematrix gebunden sind; und Polymerketten mit chromatographisch aktiven Zentren, wobei die Polymerketten an die polymeren Abstandshalterelemente gebunden sind und wobei die Polymerketten Polyacrylate oder Polymethacrylate sind. Unter dem Begriff "Chromatographiematrix" wird im Sinne der vorliegenden Erfindung jegliches Material verstanden, dass für chromatographische Verfahren als Trägermaterial der stationären Phase verwendet werden kann. Die Chromatographiematrix der vorliegenden Erfindung unterliegt keiner besonderen Einschränkung, solange sie an ihrer Oberfläche funktionelle Gruppen aufweist, an welche die polymeren Abstandshalterelemente gebunden werden können bzw. gebunden sein können. Demzufolge kann erfindungsgemäß entweder eine Chromatographiematrix eingesetzt werden, die originär funktionelle Gruppen aufweist (beispielsweise Polyesterfasern), oder eine Chromatographiematrix, bei der durch eine dem Fachmann bekannte Oberflächenmodifikation funktionelle Gruppen eingeführt wurden. Bekannte Oberflächenmodifikationen sind in diesem Zusammenhang beispielsweise eine Aktivierung mittels Plasmabehandlung, e-Beam (Elektronenstrahlbehandlung), Gamma-Bestrahlung, Hydrolyse, Aminolyse, Oxidation, Reduktion, Umsetzung mit funktionellen Carbenen und/oder Nitrenen usw.

Gemäß einer Ausführungsform der vorliegenden Erfindung weist die Chromatographiematrix 1,5 bis 30 funktionelle Gruppen pro $nm^2$ spezifische Oberfläche der Chromatographiematrix auf, d.h. die funktionelle Gruppendichte beträgt vorzugsweise von 1,5 bis 30 $nm^{-2}$ bzw. von 1,5 bis 30 $1/nm^2$. Der Begriff "spezifische Oberfläche" umfasst erfindungsgemäß die Gesamtheit aller in der Chromatographiematrix enthaltenen Oberflächen, d.h. auch jene, die sich innerhalb von vorhandenen Poren befinden. Die funktionelle Gruppendichte bezogen auf die spezifische Oberfläche lässt sich aus der spezifischen Oberfläche des Chromatographiemediums und der funktionelle Gruppendichte bezogen auf die Masse des Chromatographiemediums berechnen. Sind die funktionellen Gruppen der Chromatographiematrix Amingruppen, so wird erfindungsgemäß die Gruppendichte als "Amingruppendichte" bezeichnet.

Gemäß einer Ausführungsform der vorliegenden Erfindung umfasst die Chromatographiematrix ein Material, ausgewählt aus der Gruppe, bestehend aus natürlichen oder synthetischen Fasern, Polymer-Membranen, Polymergelen, Folien, Vliesen und Geweben. Beispielhaft für natürliche oder synthetische Fasern, die als Material für die Chromatographiematrix des erfindungsgemäßen Adsorptionsmediums verwendet werden können, können Polyesterfasern (beispielsweise "Winged Fasern" bzw. "Winged Fibers" der Firma Allasso Industries aus Polybutylenterephthalat (PBT) oder "4DG™ Fasern" der Firma Fiber Innovation Technology aus Polyethylenterephthalat (PET)) genannt werden, sowie Fasern, die Cellulose, Cellulosederivate, Nylon, Polyethylen (PE), Polyamid (PA), Polyethersulfon (PES), Polyvinylidendifluorid (PVDF), Polytetrafluorethylen (PTFE), Polypropylen (PP) und Polysulfon als strukturgebenden Bestandteil umfassen, wobei diese Materialien einzeln oder in entsprechender Kombination Anwendung finden können. Beispielhaft für Polymer-Membranen, die als Material für die Chromatographiematrix des erfindungsgemäßen Adsorptionsmediums verwendet werden können, können Membranen genannt werden, die Cellulose, Cellulosederivate, Nylon, Polyester, Polyethylen (PE), Polyamid (PA), Polyethersulfon (PES), Polyvinylidendifluorid (PVDF), Polytetrafluorethylen (PTFE), Polypropylen (PP) und Polysulfon als strukturgebenden Bestandteil umfassen, wobei diese Materialien einzeln oder in entsprechender Kombination Anwendung finden können. Beispielhaft für Polymergele, die als Material für die Chromatographiematrix des erfindungsgemäßen Adsorptionsmediums verwendet werden können, können Agarose, Dextran,

Cellulose, Polymethacrylate, Polyvinylether, Polyacrylamide, Polystyrol-Divinylbenzol-Copolymere, Silica-Dextran, Agarose-Acrylamide und Dextran-Acrylamide genannt werden. Beispielhaft für Folien und Gewebe können Folien und Gewebe aus den vorstehend genannten Polymermaterialien, welche für die Polymer-Membranen verwendet werden können, genannt werden. Beispielhaft für Vliese, die als Material für die Chromatographiematrix des erfindungsgemäßen Adsorptionsmediums verwendet werden können, können Polyester/Polyamid-Vliese (beispielsweise "Pluratexx2317 S" der Firma Freudenberg), sowie die vorstehenden Polymermaterialien, welche für die Polymer-Membranen verwendet werden können, genannt werden. Beispielhaft für anorganische Materialien, die als Material für die Chromatographiematrix des Adsorptionsmediums verwendet werden können, können Silicium, Gläser, Metallsubstrate wie Titan, Aluminiumoxid, Gold und Silber, sowie Legierungen genannt werden.

Im erfindungsgemäßen Adsorptionsmedium sind polymere Abstandshalterelemente an die Oberfläche der Chromatographiematrix gebunden, wobei die Bindung zwischen der Oberfläche der Chromatographiematrix und den Abstandshalterelementen vorzugsweise über die (originär vorhandenen oder durch Oberflächenmodifikation erzeugten) funktionellen Gruppen der Chromatographiematrix erfolgt bzw. erfolgt ist. Durch die polymeren Abstandshalterelemente, die im erfindungsgemäßen Adsorptionsmedium als Verbindungseinheiten zwischen der Chromatographiematrix und den Polymerketten mit chromatographisch aktiven Zentren (Liganden) dienen, weist das erfindungsgemäße Adsorptionsmedium eine höhere Flexibilität in den Polymerketten mit Liganden auf, was vorteilhafterweise die Zugänglichkeit dieser Polymerketten für große Zielmoleküle signifikant erhöht, da ein größeres Bindungsvolumen zur Verfügung gestellt wird. Daher werden vorteilhafterweise bei einer Bindungskonkurrenz zwischen kleinen und großen Zielmolekülen die kleineren Moleküle nicht bevorzugt. Zudem werden die chromatographisch aktiven Zentren (Liganden) optimal ausgenutzt, was vorteilhafterweise in einem größeren Wert für das Verhältnis von statischer Proteinbindungskapazität und ionischer Kapazität als bei bisher bekannten Adsorptionsmedien resultiert. Das vorstehende Verhältnis ist ein inhärenter Indikator für die Einsparung an Liganden bei der Herstellung des Adsorptionsmediums, da ein hoher Wert des Verhältnisses bedeutet, dass auch bei geringer Ligandendichte bereits eine hohe Proteinbindungskapazität erreicht wird, was im Hinblick auf die Einsparung von insbesondere teuren Liganden vorteilhafterweise die Herstellungskosten für das erfindungsgemäße Adsorptionsmedium senkt.

Unter dem Begriff "polymere Abstandshalterelemente" bzw. "polymere Spacerelemente" (kurz: "Abstandshalter" bzw. "Spacer") werden im Sinne der vorliegenden Erfindung jegliche Polymere verstanden, welche die Oberfläche der Chromatographiematrix mit den Polymerketten mit chromatisch aktiven Zentren verbinden können. Die polymeren Abstandshalterelemente der vorliegenden Erfindung unterliegen keiner besonderen Einschränkung, solange sie (bevorzugt) chemisch, aber auch physikalisch an die Oberfläche der Chromatographiematrix gebunden werden können. Gemäß einer bevorzugten Ausführungsform verfügen die polymeren Abstandshalterelemente über nucleophile funktionelle Gruppen, da diese im bevorzugten erfindungsgemäßen Herstellungsverfahren die Anbindung eines ATRP-Initiators ermöglichen.

Gemäß einer bevorzugten Ausführungsform sind die polymeren Abstandshalterelemente ausgewählt aus der Gruppe, bestehend aus Polyaminen, Polyalkoholen, Polythiolen, Polyacrylaten, Polymethacrylaten, Poly(meth)acrylamiden, Poly-N-Alkyl(meth)acrylamiden sowie Copolymeren, bestehend aus zwei oder mehr der vorstehenden Polymere, und Copolymeren, bestehend aus einem oder mehreren der vorstehenden Polymere und aus Polymeren, die keine nucleophilen funktionellen Gruppen tragen.

Polyamine im Sinne der vorliegenden Erfindung sind beispielsweise Polyallylamin, Polyvinylamin, Polyethylenimin (verzweigt oder linear), Poly(4-aminostyrol), Chitosan, Poly-L-lysin, Poly(N-methylvinylamin), Poly(N-methylallylamin), Poly(N,N-dimethylvinylamin), Poly(N,N-dimethylallylamin) und Poly(oleylamine).

Polyalkohole im Sinne der vorliegenden Erfindung sind beispielsweise Polyallylalkohol, Polyvinylalkohol, Polysaccharide (Homo- und Heteroglycane), Poly(4-hydroxystyrol) und Poly(EVOH) (EVOH = Ethylen-Vinylalkohol-Copolymer).

Polythiole im Sinne der vorliegenden Erfindung sind beispielsweise Polyvinylthiol und Polyallylthiol.

Erfindungsgemäß wird der Begriff "Poly(meth)acrylat" als Kurzform für "Polyacrylat oder Polymethacrylat" verwendet. Poly(meth)acrylate im Sinne der vorliegenden Erfindung sind beispielsweise Poly(1-glycerol(meth)acrylat), Poly(2-glycerol(meth)acrylat), Poly(hydroxyethyl(meth)acrylat), Poly(hydroxypropyl-(meth)acrylat), Poly(2-aminoethyl(meth)acrylat), Poly(2-aminopropyl(meth)acrylat), Poly(2-(diethylamino)ethyl(meth)acrylat), Poly(ethylenglycol)(meth)acrylat, Poly(hydroxybutyl(meth)acrylat), Poly(glycosyloxyethyl(meth)acrylat), Poly(3-(acryloyloxy)-2-hydroxypropyl(meth)acrylat), Poly(3-chlor-2-hydroxypropyl-(meth)acrylat), Poly([tris(hydroxymethyl)methyl](meth)acrylat), Poly(2-(4-benzoyl-3-hydroxyphenoxy)ethyl(meth)acrylate).

Sämtliche der vorstehend genannten Poly(meth)acrylate können erfindungsgemäß auch in ihrer analogen Form als Poly(meth)acrylamide und Poly-N-alkyl(meth)acrylamide verwendet werden.

Polymere, die keine nucleophilen funktionellen Gruppen tragen, sind im Sinne der vorliegenden Erfindung beispielsweise Polyolefine wie Polyethylen, Polyfluorethylen, Polypropylen, oder Polymethylmethacrylat.

Gemäß einer besonders bevorzugten Ausführungsform sind die polymeren Abstandshalterelemente Polyamine, insbesondere Polyallylamin, Polyvinylamin und/oder Polyethylenimin.

Gemäß einer weiteren Ausführungsform weisen die polymeren Abstandshalterelemente keine Epoxygruppen auf, da diese die Flexibilität der Abstandshalterelemente und damit auch die Flexibilität der Polymerketten mit chromatographisch aktiven Liganden durch mögliche intermolekulare Vernetzungsreaktionen einschränken können.

Gemäß einer Ausführungsform der vorliegenden Erfindung weisen die polymeren Abstandshalterelemente ein Molekulargewicht pro Element von 5.000 bis 2.000.000 g/mol auf, bevorzugt von 5.000 bis 50.000 g/mol.

Im erfindungsgemäßen Adsorptionsmedium sind die polymeren Abstandshalterelemente sowohl an die Oberfläche der Chromatographiematrix als auch an die Polymerketten mit chromatographisch aktiven Zentren (Liganden) gebunden.

Unter dem Begriff "Polymerketten mit chromatographisch aktiven Zentren" werden im Sinne der vorliegenden Erfindung Polymerketten verstanden, die chromatographisch aktive Zentren (Liganden) aufweisen. Die Polymerketten an sich, d.h. die Polymerketten ohne chromatographisch aktiven Zentren, sind Polyacrylate oder Polymethacrylate. Besonders bevorzugt ist dabei beispielsweise Glycidyl(meth)acrylat, da das erfindungsgemäße Adsorptionsmedium in diesem Fall aufgrund der Polymerisierbarkeit der Poly(meth)acrylate über ATRP einfach synthetisch zugänglich ist.

Gemäß der vorliegenden Erfindung sind "chromatographisch aktive Zentren" bzw. "Liganden" Stellen bzw. Gruppen in bzw. an den Polymerketten, die befähigt sind, mit in Fluiden enthaltenen Adsorbenden Wechselwirkungen einzugehen. Die chromatographisch aktiven Zentren im erfindungsgemäßen Adsorptionsmedium unterliegen keiner besonderen Einschränkung. Gemäß einer Ausführungsform der vorliegenden Erfindung sind die chromatographisch aktiven Zentren der Polymerketten ausgewählt aus der Gruppe, bestehend aus anionischen und kationischen Gruppen, hydrophoben Gruppen, Affinitätsliganden, Metallchelaten und reaktiven Epoxid-, Aldehyd-, Azlacton-, N-Hydroxysuccinimid- und/oder Carbodiimid-Gruppen.

Der Begriff "Pfropfgrad" wird erfindungsgemäß durch die nachstehende Gleichung (1) beschrieben:

Pfropfgrad [%] = (Gewicht des Adsorptionsmediums mit Polymerketten – Gewicht des Adsorptionsmediums ohne Polymerketten) / (Gewicht des Adsorptionsmediums ohne Polymerketten) * 100%

(Gleichung 1)

Gemäß einer bevorzugten Ausführungsform weist das erfindungsgemäße Adsorptionsmedium einen Pfropfgrad von 1% bis 50% auf, besonders bevorzugt von 5 bis 20%. Bei einem Pfropfgrad von über 50% kann nachteilig die Permeabilität des erfindungsgemäßen Adsorptionsmediums sinken.

Des Weiteren stellt die vorliegende Erfindung ein Verfahren zur Herstellung des erfindungsgemäßen Adsorptionsmediums bereit. Die vorstehenden Ausführungen bezüglich des erfindungsgemäßen Adsorptionsmediums treffen daher auch auf das erfindungsgemäße Herstellungsverfahren zu.

Das erfindungsgemäße Verfahren zur Herstellung eines Adsorptionsmediums umfasst die Schritte:

(a) Bereitstellen einer Chromatographiematrix;
(b) Immobilisieren von polymeren Abstandshalterelementen auf der Oberfläche der Chromatographiematrix; und
(c) Immobilisieren von Polymerketten mit chromatographisch aktiven Zentren auf den polymeren Abstandshalterelementen, wobei die Polymerketten Polyacrylate oder Polymethacrylate sind.

Im Schritt (a) des erfindungsgemäßen Verfahrens wird eine wie vorstehend beschriebene Chromatographiematrix bereitgestellt, die originär funktionelle Gruppen aufweist (beispielsweise Polyesterfasern) oder bei welcher durch Oberflächenmodifikation funktionelle Gruppen eingeführt wurden.

Im Schritt (b) des erfindungsgemäßen Verfahrens werden polymere Abstandshalterelemente auf der Oberfläche der Chromatographiematrix immobilisiert, d.h. die Abstandshalterelemente werden (bevorzugt) chemisch oder auch gegebenenfalls physikalisch an die Oberfläche der Chromatographiematrix über deren funktionelle Gruppen gebunden. Der Schritt des Immobilisierens unterliegt erfindungsgemäß keiner besonderen Einschränkung und sämtliche dem Fachmann bekannte Immobilisierungsverfahren wie beispielsweise Aminolysen, Amidkupplungsreaktionen, Veresterungen, reduktive Aminierungen und Insertionsreaktionen können zur Anwendung kommen.

Im Schritt (c) des erfindungsgemäßen Verfahrens werden Polymerketten mit chromatographisch aktiven Zentren auf den polymeren Abstandshalterelementen immobilisiert, d.h. die Polymerketten mit chromatographisch aktiven Zentren werden an die polymeren Abstandshalterelemente chemisch gebunden. Der Schritt des Immobilisierens unterliegt erfindungsgemäß keiner besonderen Einschränkung und sämtliche dem Fachmann bekannten Immobilisierungsverfahren können zur Anwendung kommen. Der Schritt (c) des erfindungsgemäßen Verfahrens ist dabei so zu verstehen, dass entweder Polymerketten, die bereits chromatographisch aktive Zentren aufweisen, auf den polymeren Abstandshalterelementen immobilisiert werden, oder zuerst Polymerketten ohne chromatographisch aktive Zentren auf den polymeren Abstandshalterelementen immobilisiert werden und in diese Polymerketten dann anschließend chromatographisch aktive Zentren eingeführt werden. Verfahren zum Einführen von chromatographisch aktiven Zentren in Polymerketten sind dem Fachmann aus dem Stand der Technik bekannt (siehe beispielsweise X. Qian et al., Applied Surface Science 271 (2013) 240-247 und G. T. Hermanson et al., Immobilized Affinity Ligand Techniques, Academic Press, INC, 1992).

Gemäß einer bevorzugten Ausführungsform der vorliegenden Erfindung umfasst der Schritt (c) die Schritte:

(c1) Immobilisieren eines Polymerisationsinitiators auf den polymeren Abstandshalterelementen;
(c2) Durchführen einer Abstandshalterelement-gebundenen Polymerisation eines Monomers zum Bilden von immobilisierten Polymerketten auf den polymeren Abstandshalterelementen; und
(c3) Modifizieren der immobilisierten Polymerketten zum Bilden von chromatographisch aktiven Zentren auf den Polymerketten.

Im Schritt (c1) des Verfahrens wird ein Polymerisationsinitiator auf den polymeren Abstandshalterelementen immobilisiert, d.h. ein Polymerisationsinitiator wird chemisch an die polymeren Abstandshalterelemente gebunden. Dies wird bevorzugt über nucleophile funktionelle Gruppen der Abstandshalterelemente realisiert, und derartige Reaktionen sind dem Fachmann aus dem Stand der Technik bekannt. Der Polymerisationsinitiator unterliegt keiner besonderen Einschränkung. Beispielsweise kann als Polymerisationsinitiator $\alpha$-Bromisobutyrylbromid, 2-Brompropionsäure, Bromessigsäure, Methyl-$\alpha$-bromisobutyrat, 2-Brom-2-methylpropionsäure, Chloressigsäure, 2-Azidoethyl-2-bromisobutyrat, 3-Butynyl-2-bromisobutyrat, Propargyl-2-bromisobutyrat oder 2-Bromisobuttersäureanhydrid verwendet werden. Gemäß einer bevorzugten Ausführungsform ist der Polymerisationsinitiator $\alpha$-Bromisobutyrylbromid.

Die Initiatordichte, d.h. die Anzahl der immobilisierten Polymerisationsinitiatormoleküle pro $nm^2$ spezifische Oberfläche der Chromatographiematrix, beträgt vorzugsweise nach dem Schritt (c1) des erfindungsgemäßen Verfahrens von 1,5 bis 30 $nm^{-2}$ bzw. von 1,5 bis 30 $1/nm^2$.

Im Schritt (c2) des Verfahrens wird eine Abstandshalterelement-gebundene Polymerisation eines Monomers durchgeführt, wodurch immobilisierte Polymerketten auf den polymeren Abstandshalterelementen gebildet werden. Gemäß einer besonders bevorzugten Ausführungsform wird die Abstandshalterelement-gebundene Polymerisation im Schritt (c2) mittels einer dem Fachmann bekannten Atomtransferradikalpolymerisation (ATRP) mit einer Polymerisationslösung durchgeführt. Auf diese Weise kann gewährleistet werden, dass die Polymerketten einen sehr geringen Vernetzungs- und/oder Verzweigungsgrad aufweisen, da ungewollte Nebenreaktionen wie "back-biting" (Intramolekulare Cyclisierungen von wachsenden Polymerketten), Übertragungsreaktionen usw. unterdrückt werden können. Sehr geringe Vernetzungs- und/oder Verzweigungsgrade der Polymerketten gewährleisten vorteilhafterweise eine hohe Flexibilität der Polymerketten und damit auch eine hervorragende Zugänglichkeit der chromatographisch aktiven Zentren.

Gemäß einer besonders bevorzugten Ausführungsform des erfindungsgemäßen Verfahrens wird im Schritt (c2) eine Polymerisationslösung verwendet, die einen Anteil von höchstens 3 mol-% an bifunktionellen Monomeren, bezogen auf die Gesamtmenge der Monomere in der Lösung, aufweist. Unter dem Begriff "bifunktionelle Monomere" werden erfindungsgemäß insbesondere Monomere verstanden, die als vernetzungsfähige Gruppen Epoxide, Halogenide, Isocyanate oder mehrere polymerisierbare Funktionalitäten aufweisen. Ein Anteil von höchstens 3 mol-% an bifunktionellen Monomeren in der Polymerisationslösung, bezogen auf die Gesamtmenge der Monomere in der Lösung, führt vorteilhafterweise zu einer besonders hohen Flexibilität der Polymerketten mit chromatographisch aktiven Liganden, da nur eine geringe Vernetzung der Polymerketten erzeugt wird. Demzufolge weist die Polymerisationslösung im Schritt (c2) bevorzugt höchstens 1,5 mol-% an bifunktionellen Monomeren, bezogen auf die Gesamtmenge der Monomere in der Lösung, auf, besonders bevorzugt höchstens 0,75 mol-% und am meisten bevorzugt 0 mol-%, d.h. keine bifunktionellen Monomere sind in der Polymerisationslösung vorhanden.

Im Schritt (c3) des Verfahrens werden die immobilisierten Polymerketten modifiziert, um so chromatographisch aktive Zentren auf bzw. an den Polymerketten zu bilden. Verfahren zum Einführen von chromatographisch aktiven Zentren in Polymerketten sind dem Fachmann aus dem Stand der Technik bekannt. Beispielsweise können mit Natriumsulfit in den Polymerketten vorhandene Epoxygruppen sulfoniert und damit zu einem Kationentauscher umgesetzt werden, oder mit Trimethylamin zu quartären Ammoniumgruppen und damit zu einem Anionentauscher umgesetzt werden. Weisen die im Schritt (c2) auf den polymeren Abstandshalterelementen immobilisierten Polymerketten bereits chromatographisch aktive Zentren auf, so kann auf den optionalen Schritt (c3) verzichtet werden.

Des Weiteren stellt die vorliegende Erfindung die Verwendung des erfindungsgemäßen Adsorptionsmediums oder eines gemäß des erfindungsgemäßen Verfahrens hergestellten Adsorptionsmediums zur Aufreinigung von Biomolekülen bereit. Bei den aufzureinigenden Biomolekülen handelt es sich erfindungsgemäß um Proteine, Peptide, Aminosäuren, Nukleinsäuren, Virus-artige Partikel, Viren und/oder Endotoxine.

Durch die polymeren Abstandshalterelemente weist das erfindungsgemäße Adsorptionsmedium vorteilhafterweise eine höhere Flexibilität in den Polymerketten mit Liganden auf, was die Zugänglichkeit dieser Polymerketten für

große Zielmoleküle signifikant erhöht, da ein größeres Bindungsvolumen zur Verfügung gestellt wird. Daher werden vorteilhafterweise bei einer Bindungskonkurrenz zwischen kleinen und großen Zielmolekülen die kleineren Moleküle nicht bevorzugt. Zudem werden die chromatographisch aktiven Zentren (Liganden) optimal ausgenutzt, was vorteilhafterweise in einem größeren Wert für das Verhältnis von statischer Proteinbindungskapazität und ionischer Kapazität als bei bisher bekannten Adsorptionsmedien resultiert. Demzufolge kann im erfindungsgemäßen Adsorptionsmedium die Zahl der Liganden bzw. der Polymerketten mit Liganden reduziert werden, was einerseits in einer kostengünstigeren Herstellung resultiert und andererseits die Permeabilität des Adsorptionsmaterials nicht nachteilig vermindert. Das erfindungsgemäße Verfahren stellt vorteilhafterweise dieses Adsorptionsmedium zuverlässig bereit, d.h. die hohe Flexibilität der Polymerketten mit Liganden kann auf geeignete Weise über das erfindungsgemäße Verfahren, insbesondere mittels einer ATRP-Reaktion, realisiert werden. Das erfindungsgemäße Adsorptionsmaterial eignet sich daher hervorragend zur Aufreinigung von Biomolekülen, woran ein großes industrielles Interesse besteht. Die vorliegende Erfindung wird anhand der folgenden, nicht einschränkenden Beispiele näher erläutert.

## Beispiele

Methoden:

*Bestimmung der Amingruppendichte über photometrische Bestimmung der Orange II Bindung:*

Die Bestimmung der Amingruppendichte erfolgte nach einer Methode von NOEL et al. (Bioconjugate Chemistry 22 (2011) 1690-1699) an PET-Fasern, welche zuvor einer Aminolysereaktion unterzogen wurden. Dazu wurden die gewogenen Faserproben in einer Farbstofflösung (1,4 mg/ml Orange II in 1 mM HCl) geschwenkt (40 °C, 30 min), dreimal in 1 mM HCl ausgespült (RT, 10 min), um nicht gebundenen Farbstoff zu entfernen, und anschließend getrocknet (10 min, 80 °C).

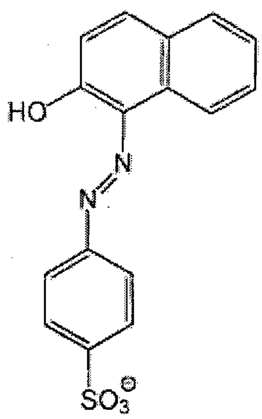

Orange II

Anschließend wurden die Proben in verdünnter Natronlauge (20 mL, pH = 12) geschwenkt (40 °C, 30 min), um eine Deprotonierung der Amingruppen zu erreichen und die ionisch gebundenen Farbstoffmoleküle zu eluieren. Nach Entnahme der Fasern wurde die Lösung mit Salzsäure (200 µL, 32 %) auf pH = 3. eingestellt und gegebenenfalls mit 1 mM Salzsäure verdünnt.

Von der so erhaltenen Lösung wurde die Absorbanz bei einer Wellenlänge von 484 nm bestimmt. Unter Kenntnis des molaren Extinktionskoeffizienten des Orange II Farbstoffs und des Verdünnungsfaktors kann auf die Stoffmenge der Farbstoffmoleküle in der Lösung geschlossen werden. Da jede protonierte Amingruppe in der Lage ist, ein Orange II Molekül unter sauren Bedingungen zu binden, kann so indirekt auf die Zahl der Amingruppen auf der Faserprobe geschlossen werden.

*Bestimmung der ionischen Kapazität über Säure-Base-Titration:*

Zur Bestimmung der ionischen Kapazität mittels Säure-Base-Titration wird eine (Faser-)Probe mit bekanntem Gewicht trocken in eine Säule gepackt und auf eine Dichte von 0,35 g/mL komprimiert. Die Titration umfasst folgende Schritte:

- Equlibrieren und Benetzen mit 10 mM KPi-Puffer (pH = 7, 15 Bettvolumen)
- Vollständige Protonierung der Sulfonsäuregruppen mittels 5 % HCl (20 Bettvolumen)
- Spülen mit demineralisiertem Wasser (15 Bettvolumen)
- Titration mit 10 mM NaOH Lösung (20 Bettvolumen)
- Spülen mit 10 mM KPi-Puffer (pH = 7,15 Bettvolumen)

Während der Titration wird das Leitfähigkeitssignal am Säulenausgang verfolgt. Unter Kenntnis des Totvolumens der Säule und Anlage kann aus der Durchbruchskurve der 10 mM NaOH-Lösung mittels Integration auf die ionische Kapazität geschlossen werden, wie anhand von Figur 1 erläutert wird:
Unter Kenntnis der Fläche $A_2$, welche mittels einer einfachen Integration zugänglich ist, kann auf die Fläche $A_1$ geschlossen werden. Die ionische Bindungskapazität bezogen auf die Fasermasse lässt sich wie folgt berechnen:

$$\text{ion. Bindungskapazität}\left[\frac{\text{mmol}}{\text{g}}\right] = \frac{A_2\left[\frac{\text{mS}}{\text{cm}}\cdot\text{L}\right]\cdot 10\,\frac{\text{mmol}}{\text{L}}}{C_0\left[\frac{\text{mS}}{\text{cm}}\right]\cdot m_{Fasern}[\text{g}]}$$

*Bestimmung der Hydroxylgruppendichte:*

Zur Bestimmung der Hydroxylgruppendichte wurde die jeweilige Substratprobe mit Natrium-3-brompropansulfonat umgesetzt. Dazu wurden 0,5 g Substrat in 5 mL einer Lösung, bestehend aus 50,2 wt.-% RO-Wasser, 29,8 wt.-% einer 32 wt.-% NaOH-Lösung und 20 wt.-% Natrium-3-brompropansulfonat, für 24 h inkubiert. Die erhaltenen Fasern wurden im Anschluss mit RO-Wasser gewaschen.

Die erhaltene ionische Kapazität wurde wie oben beschrieben mittels Säure-Base-Titration ermittelt und unter Annahme eines vollständigen Umsatzes mit der Zahl an Hydroxylgruppen gleichgesetzt.

*Initiatordichtebestimmung:*

Zur Bestimmung der Menge an immobilisiertem Initiator auf den aminolysierten Proben wurde eine von KOSIOL (Masterarbeit P. Kosiol, Georg-August-Universität Göttingen, 2013) entwickelte Methode eingesetzt.

Reagenzien:

- Puffer: Natriumacetat (68 g) wurde in 200 mL Wasser gelöst und Eisessig (30 mL) hinzugegeben. Die Lösung wurde mit Wasser auf 1 L aufgefüllt. Der pH-Wert wurde auf 4,7 eingestellt.
- Phenolrot-Lösung: Phenolrot (120 mg) wurde in 200 mL Wasser gelöst. 0,1 M NaOH (12 mL) wurden hinzugegeben und die Lösung auf 1 L aufgefüllt.
- Chloramin-T-Lösung: Chloramin-T (0,15 g) wurde auf 100 mL aufgefüllt. Die Lösung wurde täglich frisch angesetzt.
- Natriumthiosulfat-Lösung: Natriumthiosulfat (2,5 g) wurde auf 100 mL aufgefüllt.
- Kalibrationsstammlösung: Kaliumbromid (74,5 mg) wurde auf 500 mL aufgefüllt. Die Konzentration an Bromid betrug 0.01 g/L.
- Kalibrationsstandards: Durch Verdünnung mit Wasser wurden nach dem folgenden Pipettierschema unterschiedlich konzentrierte Kalibrationsstandards aus der Stammlösung hergestellt. Von diesen Standards wurden jeweils 5 mL weiter verwendet.

Tabelle 1: Pipettierschema zur Herstellung wässriger Kalibrierstandards

| Volumen der Stammlösung / µL | Endvolumen des Kalibrierstandards / mL | Masse Bromid in 5 mL Lösung / µg |
|---|---|---|
| **200** | 10 | 10 |
| **400** | 10 | 20 |

(fortgesetzt)

| **Volumen der Stammlösung / µL** | **Endvolumen des Kalibrierstandards / mL** | **Masse Bromid in 5 mL Lösung / µg** |
|---|---|---|
| **800** | 10 | 40 |
| **1200** | 10 | 60 |
| **1600** | 10 | 80 |
| **2000** | 10 | 100 |

Erstellung einer Kalibration

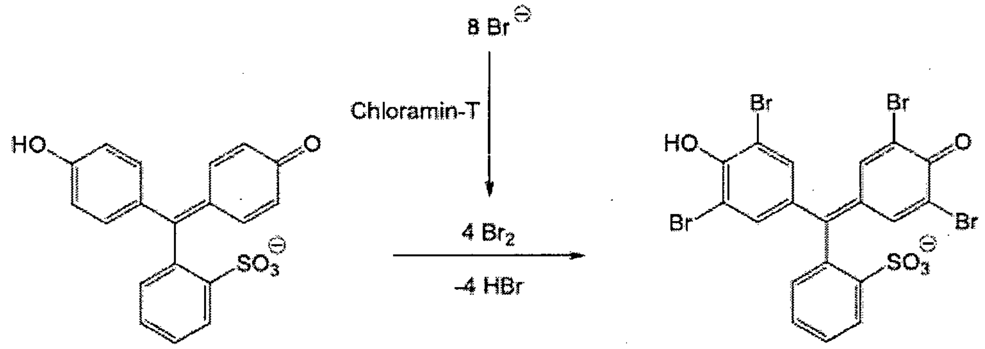

Phenolrot
$\lambda_{max}$ = 356 nm
Bromphenolblau
$\lambda_{max}$ = 589 nm

5,00 mL des jeweiligen Kalibrierstandards wurden in 10 mL Maßkolben mit 2,00 mL Pufferlösung und 1,25 mL Phenolrot-Lösung versetzt. Unter Schütteln wurden 300 µL Chloramin-T-Lösung dazugegeben. Nach genau 1 min wurden 500 µL Thiosulfat-Lösung dazugegeben. Die Lösung wurde auf 10 mL aufgefüllt. Über das entstandene Bromphenolblau wurde die Absorbanz bei 585 nm gemessen, die linear mit der Konzentration an Bromphenolblau zusammenhängt. Als Blindwert diente eine Probe, bei der statt des Kalibrationsstandards die gleiche Menge an Wasser zugegeben wurde. Die Kalibration erfolgte als Doppelbestimmung mit zwei Sätzen von Kalibrationsstandards.

Mittels der Kalibriergeraden aus Figur 2 konnte folgender Zusammenhang zwischen der Absorbanz A und der in der Probe befindlichen Masse an Bromid $m_{Br^-}$ aufgestellt werden:

$$m_{Br^-}[\mu g] = \left(\frac{A + 0{,}0204}{0{,}0185}\right)$$

Probenvorbereitung - Dehalogenierung eines immobilisierten Initiators

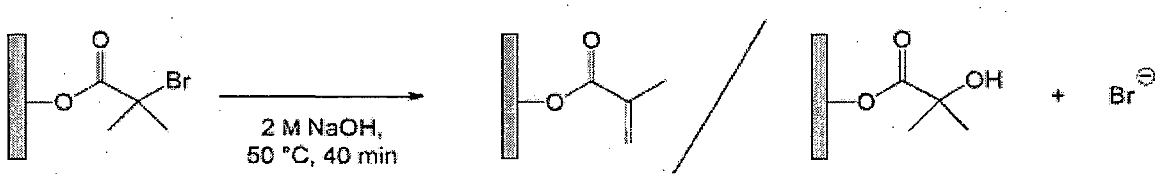


Mit Initiator versehene Proben wurden gewogen. Anschließend wurden die Proben in 25 mL Laborflaschen überführt. 20 mL 2 M Natronlauge wurde jeweils hinzugegeben und die Suspensionen 40 min bei 50 °C gerührt. Die gewählten Reaktionsbedingungen ermöglichten eine quantitative Dehalogenierung des Initiators. Die Lösungen wurden auf Raumtemperatur abgekühlt und mit Hilfe von Spritzenvorsatzfiltern (0,20 µm) filtriert. 10 mL der Lösungen wurden jeweils in 20 mL Maßkolben überführt und jeweils mit einem Tropfen Phenolrot-Lösung versetzt. Die Lösungen wurden

mit 1,5 M Schwefelsäure bis zu einem Farbumschlag nach gelb angesäuert. Danach wurde mit Wasser auf 20 mL aufgefüllt und so die aufbereiteten Probenlösungen erhalten.

Zur Bestimmung der Bromidmenge einer Probe mit unbekanntem Bromidgehalt wurden von jeder aufbereiteten Probenlösung die in Tabelle 2 dargestellten Verdünnungen hergestellt. Die Verdünnungen erfolgten in 10 mL Maßkolben. Durch Pipettieren von Probenlösung und Wasser wurden stets Volumina von 5 mL eingestellt. Anschließend wurden die verdünnten Probenlösungen analog zu den Kalibrierstandards umgesetzt und die Absorbanzwerte ermittelt.

Tabelle 2: Pipettierschema zur Herstellung von verdünnten Probenlösungen

| # | Volumen der aufbereiteten Probenlösung / mL | Volumen Wasser / mL | Endvolumen / mL | Verdünnungsfaktor |
|---|---|---|---|---|
| **1** | 0,10 | 4,90 | 10 | 0,0025 |
| **2** | 0,25 | 4,75 | 10 | 0,00625 |
| **3** | 0,50 | 4,50 | 10 | 0,0125 |
| **4** | 1,0 | 4,00 | 10 | 0,025 |
| **5** | 2,0 | 3,00 | 10 | 0,05 |
| **6** | 5,0 | 0,00 | 10 | 0,125 |

Über die vorstehende Formel konnte die Masse an Bromid in der Probe berechnet werden. Die Gesamtverdünnungsfaktoren zur Umrechnung der Bromidmasse auf die gesamte Membran sind Tabelle 2 zu entnehmen. Nur die Absorbanzwerte konnten weiter verwendet werden, die untereinander einen linearen Zusammenhang mit den jeweiligen Verdünnungsfaktoren zeigten. Abweichungen vom linearen Verhalten waren unter anderem dadurch bedingt, dass das Verhältnis der eingesetzten Reagenzien zu den Bromidmengen nicht konstant war. Bei großen Mengen an Bromid in der Probe beispieisweise führte der Überschuss an gebildetem molekularem Brom gegenüber der konstanten Menge an Phenolrot zu einer Zerstörung der nachzuweisenden Tetrabrom-Verbindung. Die daraus resultierenden Absorbanzwerte wichen vom linearen Verhalten negativ ab.

Synthesebeschreibungen:

Beispiel 1: Modifikation von Polyesterfasern (PBT und PET) mit Abstandshalterelement

*Abstandshalterimmobilisierung:*

In einer typischen Umsetzung wurde eine 10 wt.% Poly(allylamin)-Lösung in RO-Wasser (70 v/v %) und 1,4-Dioxan (30 v/v %) auf einen pH-Wert von 12,5 mittels 32 wt.% NaOH-Lösung eingestellt und mit einer abgewogenen Menge an Winged Fasern (PBT) (Alasso Industries) oder 4DG™ Fasern (PET) (Fiber Innovation Technology) versetzt. Pro Gramm Polyester-Faser wurden 50 mL Poly(allylamin)-Lösung (MW: 15.000 g/mol; Firma Beckmann-Kenko GmbH) eingesetzt.

Die Suspension wurde bei 80 °C für 6 h gerührt. Nach Erreichen der gewünschten Umsatzdauer wurden die Fasern abfiltriert und in einen großen Überschuss an RO-Wasser gegeben und für 30 min gerührt. Im Anschluss wurde erneut abfiltriert und solange mit RO-Wasser gewaschen, bis das Filtrat keine basische Reaktion mehr zeigt. Die erhaltenen aminierten Fasern wurden nach einer Trocknung für 6 h bei 60 °C bezüglich Massenverlust und Amingruppendichte charakterisiert.

| Fasertyp | Amingruppendichte |
|---|---|
| Winged Fibers | 12 ± 1,5 $nm^{-2}$ |
| 4DG™ | 18 ± 4,5 $nm^{-2}$ |

*Initiatorimmobilisierung*

In einer typischen Immobilisierung wurden α-Bromisobutyrylbromid (1,00 mL, 1,86 g, 8,09 mmol) und Triethylamin (1,50 mL, 1,08 g, 10,7 mmol) zu 100 mL wasserfreiem Dichlormethan gegeben. In dieser Lösung wurden 4 g aminierte Fasern suspendiert und 2 h bei Raumtemperatur geschüttelt. Zur Aufarbeitung wurden langsam 10 mL Isopropanol tropfenweise zugegeben und es wurde weitere 30 min geschüttelt. Im Anschluss wurden die Fasern abfiltriert und mit Wasser, Isopropanol, Aceton und wieder Wasser gewaschen. Nach einer Trocknung bei 40 °C über 12 h wurden die mit Initiator versehen Fasern bezüglich der Initiatordichte charakterisiert.

| Fasertyp | Initiatordichte |
|---|---|
| Winged Fibers | $10{,}5 \pm 1{,}5\ nm^{-2}$ |
| 4DG™ | $15 \pm 4\ nm^{-2}$ |

*Abstandshaltergebundene Polymerisation:*

In einer repräsentativen Polymerisation wurde Glycidylmethacrylat (2,5 g, 17,5 mmol, 175 Äq.) in 100 mL RO-Wasser und 100 mL Isopropanol gelöst. Die Lösung wurde im Anschluss für 30 min durch Einleiten von Stickstoff entgast. Im Stickstoffgegenstrom wurden Kupfer(I)bromid (15,0 mg, 104 μmol, 1,0 Äq.), 2,2'-Bipyridin (50,0 mg, 320 μmol, 3,1 Äq.) und Ascorbinsäure (45,0 mg, 225 μmol, 1,25 Äq.) hinzugegeben und es wurde für weitere 30 min unter Rühren entgast. Parallel wurden 1,0 g mit Initiator versehene Fasern im Stickstoffstrom entgast. Mittels einer Überführungskanüle wurde die Polymerisationslösung unter Schutzgas zu den Fasern gegeben und es wurde unter Sauerstoffausschluss für 8 h gerührt. Die Polymerisation wurde durch Öffnung des Reaktionsgefäßes und durch Einleiten von Sauerstoff terminiert. Die mit Glycidylmethacrylat modifizierten Fasern wurden mit Wasser und Aceton gewaschen um Katalysatorreste und Polymer, das nicht auf der Oberfläche gebunden ist, zu entfernen. Das Zielprodukt wurde nach Trocknen für 6 h bei 40 °C erhalten. Der Pfropfgrad wurde gravimetrisch nach Gleichung (1) bestimmt.

| Fasertyp | Pfropfgrad |
|---|---|
| Winged Fibers | 6 ± 1 % |
| 4DG™ | 2 ± 0,5 % |

*Sulfonierung der Epoxidgruppen:*

Die Umsetzung zu einem starken Kationentauscher erfolgte folgendermaßen:
600 g Wasser wurden vorgelegt und unter Kühlung 180 g $Na_2SO_3$, 20 g $Na_2HPO_4 \cdot 2\ H_2O$ und 50 g TBABS (Tetrabutylammoniumbisulfat) gelöst. Der pH-Wert der Lösung wurde mit NaOH (32 %) auf 8 eingestellt und es wurde schrittweise Wasser zugegeben, bis 1 kg Lösung erreicht waren. Dabei wurde der pH-Wert regelmäßig kontrolliert und bei Abweichung mit NaOH (32 %) auf pH 8 nachreguliert.

Zur Umsetzung wurde so viel Lösung verwendet, dass alle Fasern frei in einer 500 ml Weithalsgewindeflasche schwimmen konnten. Die Lösung wurde auf 85 °C erwärmt und die Fasern unter Rühren 45 min bei 85 °C umgesetzt.

| Trägermaterial | Ionische Kapazität |
|---|---|
| Winged Fibers | 220 ± 25 μmol/g |
| 4DG™ | 60 ± 10 μmol/g |

Beispiel 2: Modifikation von Polvester/Polvamid-Vlies mit Abstandshalterelement

*Abstandshalterimmobilisierung :*

In einer typischen Umsetzung wurde eine 10 wt.% Poly(allylamin)-Lösung in RO-Wasser (70 v/v %) und 1,4-Dioxan (30 v/v %) auf einen pH-Wert von 12,5 mittels 32 wt.% NaOH-Lösung eingestellt und mit Stanzlingen (47 mm Durchmesser) eines Splitfaser-Vlieses (Pluratexx2317 S, Firma Freudenberg) versetzt. Pro Stanzling wurden 10 mL Poly(allylamin)-Lösung (wie in Beispiel 1 beschrieben) eingesetzt.

Die Suspension wurde bei 80 °C für 6 h gerührt. Nach Erreichen der gewünschten Umsatzdauer wurden die Stanzlinge aus der Lösung entnommen und in einen großen Überschuss an RO-Wasser gegeben und solange mit RO-Wasser gewaschen, bis das Filtrat keine basische Reaktion mehr zeigt. Die erhaltenen aminierten Vliesmaterialien wurden nach einer Trocknung für 6 h bei 60 °C bezüglich Massenverlust und Amingruppendichte charakterisiert.

| Trägermaterial | Amingruppendichte |
|---|---|
| Splitfaser PET/PA6 Vlies | $9{,}0\ nm^{-2}$ |
| (PET: Polyethylenterephthalat; PA6: Polyamid 6) | |

*Initiatorimmobilisierung:*

In einer typischen Immobilisierung wurden α-Bromisobutyrylbromid (1,00 mL, 1,86 g, 8,09 mmol) und Triethylamin (1,50 mL, 1,08 g, 10,7 mmol) zu 100 mL wasserfreiem Dichlormethan gegeben. In dieser Lösung wurden 12 Stanzlinge (47 mm Durchmesser) suspendiert und 2 h bei Raumtemperatur geschüttelt. Zur Aufarbeitung wurden langsam 10 mL Isopropanol tropfenweise zugegeben und weitere 30 min geschüttelt. Im Anschluss wurden die Stanzlinge entnommen und mit Wasser, Isopropanol, Aceton und wieder Wasser gewaschen. Nach einer Trocknung bei 40 °C über 12 h wurden die mit Initiator versehen Stanzlinge bezüglich der Initiatordichte charakterisiert.

| **Fasertyp** | **Initiatordichte** |
|---|---|
| Splitfaser PET/PA6 Vlies | 7,5 $nm^{-2}$ |

*Abstandshaltergebundene Polymerisation:*

In einer repräsentativen Polymerisation wurde Glycidylmethacrylat (2,5 g, 17,5 mmol, 175 Äq.) in 100 mL RO-Wasser und 100 mL Isopropanol gelöst. Die Lösung wurde im Anschluss für 30 min durch Einleiten von Stickstoff entgast. Im Stickstoffgegenstrom wurden Kupfer(I)bromid (15,0 mg, 104 μmol, 1,0 Äq.), 2,2'-Bipyridin (50,0 mg, 320 μmol, 3,1 Äq.) und Ascorbinsäure (45,0 mg, 225 μmol, 1,25 Äq.) hinzugegeben und es wurde für weitere 30 min unter Rühren entgast. Parallel wurden 4 Stanzlinge (47 mm Durchmesser) im Stickstoffstrom entgast. Mittels einer Überführungskanüle wurde die Polymerisationslösung unter Schutzgas zu den Fasern gegeben und es wurde unter Sauerstoffausschluss für 16 h gerührt. Die Polymerisation wurde durch Öffnung des Reaktionsgefäßes und durch Einleiten von Sauerstoff terminiert. Die mit Glycidylmethacrylat modifizierten Fasern wurden mit Wasser und Aceton gewaschen um Katalysatorreste und Polymer, das nicht auf der Oberfläche gebunden ist, zu entfernen. Das Zielprodukt wurde nach Trocknen für 6 h bei 40 °C erhalten. Der Pfropfgrad wurde gravimetrisch nach Gleichung (1) bestimmt.

| **Fasertyp** | **Pfropfgrad** |
|---|---|
| Splitfaser PET/PA6 Vlies | 7 % |

*Sulfonierung der Epoxidgruppen:*

Die Umsetzung zu einem starken Kationentauscher erfolgt folgendermaßen:
600 g Wasser wurden vorgelegt und es wurden unter Kühlung 180 g $Na_2SO_3$, 20 g $Na_2HPO_4 \cdot 2\ H_2O$ und 50 g TBABS gelöst. Der pH-Wert der Lösung wurde mit NaOH (32 %) auf 8 eingestellt und es wurde schrittweise Wasser zugegeben, bis 1 kg Lösung erreicht waren. Dabei wurde der pH-Wert regelmäßig kontrolliert und bei Abweichung mit NaOH (32 %) auf pH 8 nachreguliert.

Zur Umsetzung wurde so viel Lösung verwendet, dass alle Stanzlinge frei in einer 500 ml Weithalsgewindeflasche schwimmen konnten. Die Lösung wurde auf 85 °C erwärmt und die Stanzlinge unter Rühren 45 min bei 85 °C umgesetzt.

| **Trägermaterial** | **Ionische Kapazität** |
|---|---|
| Splitfaser PET/PA6 Vlies | 2,70 $\mu mol/cm^2$ |

<u>Beispiel 3: Modifikation von Nylonmembran mit Abstandshalterelement</u>

*Abstandshalterimmobilisierung:*

In einer typischen Umsetzung wurde eine 10 wt.% Poly(allylamin)-Lösung (wie in Beispiel 1 beschrieben) in RO-Wasser (70 v/v %) und 1,4-Dioxan (30 v/v %) auf einen pH-Wert von 12,5 mittels 32 wt.% NaOH-Lösung eingestellt und mit Stanzlingen (47 mm Durchmesser) einer Nylonmembran (Typ 21303, mittlere Porengröße 1,2 μm, Firma Sartorius AG) versetzt. Pro Stanzling wurden 10 mL Poly(allylamin)-Lösung eingesetzt.

Die Suspension wurde bei 80 °C für 6 h gerührt. Nach Erreichen der gewünschten Umsatzdauer wurden die Stanzlinge aus der Lösung entnommen und in einen großen Überschuss an RO-Wasser gegeben und solange mit RO-Wasser gewaschen, bis das Filtrat keine basische Reaktion mehr zeigt. Die erhaltenen aminierten Vliesmaterialien wurden nach einer Trocknung für 6 h bei 60 °C bezüglich Massenverlust und Amingruppendichte charakterisiert.

| Trägermaterial | Amingruppendichte |
|---|---|
| Nylonmembran | 2,1 $nm^{-2}$ |

*Initiatorimmobilisierung:*

In eine Lösung aus Dichlormethan (49,5 mL) und α-Bromisobutyrylbromid (500 μL) wurden Stanzlinge (47 mm) einer mit Polyallylamin umgesetzten Nylonmembran (Typ 21303) getaucht, bis diese vollständig benetzt waren. Die benetzten Stanzlinge wurden für 5 min bei Raumtemperatur inkubiert und anschließend für 30 min in Isopropanol geschüttelt, um die Reaktion zu terminieren. Im Anschluss wurde für 20 min mit RO-Wasser gespült und bei 60 °C für 1 h getrocknet.

*Abstandshaltergebundene Polymerisation:*

In einer repräsentativen Polymerisation wurde Glycidylmethacrylat (2,5 g, 17,5 mmol, 175 Äq.) in 100 mL RO-Wasser und 100 mL Isopropanol gelöst. Die Lösung wurde im Anschluss für 30 min durch Einleiten von Stickstoff entgast. Im Stickstoffgegenstrom wurden Kupfer(I)bromid (15,0 mg, 104 μmol, 1,0 Äq.), 2,2'-Bipyridin (50,0 mg, 320 μmol, 3,1 Äq.) und Ascorbinsäure (45,0 mg, 225 μmol, 1,25 Äq.) hinzugegeben und es wurde für weitere 30 min unter Rühren entgast. Parallel wurden 4 Stanzlinge (47 mm Durchmesser) im Stickstoffstrom entgast. Mittels einer Überführungskanüle wurde die Polymerisationslösung unter Schutzgas zu den Fasern gegeben und es wurde unter Sauerstoffausschluss für 90 min gerührt. Die Polymerisation wurde durch Öffnung des Reaktionsgefäßes und durch Einleiten von Sauerstoff terminiert. Die mit Glycidylmethacrylat modifizierten Fasern wurden mit Wasser und Aceton gewaschen um Katalysatorreste und Polymer, das nicht auf der Oberfläche gebunden ist, zu entfernen. Das Zielprodukt wurde nach Trocknen für 6 h bei 40 °C erhalten. Der Pfropfgrad wurde gravimetrisch nach Gleichung (1) bestimmt.

| Fasertyp | Pfropfgrad |
|---|---|
| Nylonmembran | 2 % |

*Sulfonierung der Epoxidgruppen:*

Die Umsetzung zu einem starken Kationentauscher erfolgte folgendermaßen:
600 g Wasser wurden vorgelegt und es wurden unter Kühlung 180 g $Na_2SO_3$, 20 g $Na_2HPO_4 \cdot 2\ H_2O$ und 50 g TBABS gelöst. Der pH-Wert der Lösung wurde mit NaOH (32 %) auf 8 eingestellt und schrittweise Wasser zugegeben, bis 1 kg Lösung erreicht waren. Dabei wurde der pH-Wert regelmäßig kontrolliert und bei Abweichung mit NaOH (32 %) auf pH 8 nachreguliert.

Zur Umsetzung wurde so viel Lösung verwendet, dass alle Stanzlinge frei in einer 500 ml Weithalsgewindeflasche schwimmen konnten. Die Lösung wurde auf 85 °C erwärmt und die Stanzlinge unter Rühren 45 min bei 85 °C umgesetzt.

| Trägermaterial | Ionische Kapazität |
|---|---|
| Nylonmembran | 0,3 ± 0,02 μmol/$cm^2$ |

<u>Beispiel 4: Modifikation von PBT-Fasern mit Abstandshalterelement</u>

*Abstandshalterimmobilisierung:*

In einer typischen Umsetzung wurden Winged Fasern (PBT) für 1 h in einer Lösung, bestehend aus 30 wt.% 1,4-Butandioldiglycidylether (BuDGE) und 70 wt.% 0,15 M KOH-Lösung, bei Raumtemperatur geschüttelt. Die Fasern wurden abfiltriert und mit Ethanol und RO-Wasser gewaschen. Die erhaltenen epoxyaktivierten Fasern wurden im Anschluss für 16 h bei 80 °C in einer Lösung aus 2 wt.-% Polyvinylalkohol (PVA, $M_w$ = 9.000 - 10.000) in 0,15 M KOH-Lösung umgesetzt. Die Fasern wurden im Anschluss abfiltriert und mit Ethanol und RO-Wasser gewaschen und für 12 h bei 40 °C getrocknet.

| Fasertyp | Hydroxygruppendichte |
|---|---|
| Winged Fibers | 18,3 $nm^{-2}$ |

*Initiatorimmobilisierung:*

In einer typischen Immobilisierung wurden α-Bromisobutyrylbromid (1,00 mL, 1,86 g, 8,09 mmol) und Triethylamin (1,50 mL, 1,08 g, 10,7 mmol) zu 100 mL wasserfreiem Dichlormethan gegeben. In dieser Lösung wurden 4 g PVA-modifizierte Fasern suspendiert und 2 h bei Raumtemperatur geschüttelt. Zur Aufarbeitung wurden langsam 10 mL Isopropanol tropfenweise zugegeben und es wurde weitere 30 min geschüttelt. Im Anschluss wurden die Fasern abfiltriert und mit Wasser, Isopropanol, Aceton und wieder Wasser gewaschen. Nach einer Trocknung bei 40 °C über 12 h wurden die mit Initiator versehenen Fasern bezüglich der Initiatordichte charakterisiert.

| Fasertyp | Initiatordichte |
|---|---|
| Winged Fibers | 15,6 $nm^{-2}$ |

*Abstandshaltergebundene Polymerisation:*

In einer repräsentativen Polymerisation wurde Glycidylmethacrylat (2,5 g, 17,5 mmol, 175 Äq.) in 100 mL RO-Wasser und 100 mL Isopropanol gelöst. Die Lösung wurde im Anschluss für 30 min durch Einleiten von Stickstoff entgast. Im Stickstoffgegenstrom wurden Kupfer(I)bromid (15,0 mg, 104 μmol, 1,0 Äq.), 2,2'-Bipyridin (50,0 mg, 320 μmol, 3,1 Äq.) und Ascorbinsäure (45,0 mg, 225 μmol, 1,25 Äq.) hinzugegeben und es wurde für weitere 30 min unter Rühren entgast. Parallel wurden 1,0 g mit Initiator versehene Fasern im Stickstoffstrom entgast. Mittels einer Überführungskanüle wurde die Polymerisationslösung unter Schutzgas zu den Fasern gegeben und es wurde unter Sauerstoffausschluss für 5 h gerührt. Die Polymerisation wurde durch Öffnung des Reaktionsgefäßes und durch Einleiten von Sauerstoff terminiert. Die mit Glycidylmethacrylat modifizierten Fasern wurden mit Wasser und Aceton gewaschen, um Katalysatorreste und Polymer, das nicht auf der Oberfläche gebunden ist, zu entfernen. Das Zielprodukt wurde nach Trocknen für 6 h bei 40 °C erhalten. Der Pfropfgrad wurde gravimetrisch nach Gleichung (1) bestimmt.

| Fasertyp | Pfropfgrad |
|---|---|
| Winged Fibers | 8 % |

*Sulfonierung der Epoxidgruppen:*

Die Umsetzung zu einem starken Kationentauscher erfolgte folgendermaßen:
600 g Wasser wurden vorgelegt und es wurden unter Kühlung 180 g $Na_2SO_3$, 20 g $Na_2HPO_4 \cdot 2\,H_2O$ und 50 g TBABS gelöst. Der pH-Wert der Lösung wurde mit NaOH (32 %) auf 8 eingestellt und schrittweise Wasser zugegeben, bis 1 kg Lösung erreicht waren. Dabei wurde der pH-Wert regelmäßig kontrolliert und bei Abweichung mit NaOH (32 %) auf pH 8 nachreguliert.

Zur Umsetzung wurde so viel Lösung verwendet, dass alle Fasern frei in einer 500 ml Weithalsgewindeflasche schwimmen konnten. Die Lösung wurde auf 85 °C erwärmt und die Fasern unter Rühren 45 min bei 85 °C umgesetzt.

| Trägermaterial | Ionische Kapazität |
|---|---|
| Winged Fibers | 280 μmol/g |

Die Bindung für Lysozym betrug 305 mg/g, für IgG 278 mg/g.

Vergleichsbeispiel 1: ATRP ohne Abstandshalter auf Cellulosemembran

*Initiatorimmobilisierung:*

In eine Lösung aus Dichlormethan (49,5 mL) und α-Bromisobutyrylbromid (500 μL) wurden Stanzlinge (60 mm) einer vernetzten regenerierten Cellulosemembran (10242) getaucht, bis diese vollständig benetzt waren. Die benetzten Stanzlinge werden für 5 min bei Raumtemperatur inkubiert und anschließend für 30 min in Isopropanol geschüttelt, um die Reaktion zu terminieren. Im Anschluss wurde für 20 min mit RO-Wasser gespült und bei 60 °C für 1 h getrocknet.

| Initiatordichte |
|---|
| 10,6 $nm^{-2}$ |

*Oberflächengebundene Polymerisation:*

In einer repräsentativen Polymerisation wurde Glycidylmethacrylat (2,5 g, 17,5 mmol, 175 Äq.) in 100 mL RO-Wasser und 100 mL Isopropanol gelöst. Die Lösung wurde im Anschluss für 30 min durch Einleiten von Stickstoff entgast. Im Stickstoffgegenstrom wurden Kupfer(I)bromid (15,0 mg, 104 μmol, 1,0 Äq.), 2,2'-Bipyridin (50,0 mg, 320 μmol, 3,1 Äq.) und Ascorbinsäure (45,0 mg, 225 μmol, 1,25 Äq.) hinzugegeben und es wurde für weitere 30 min unter Rühren entgast. Parallel wurden 5 Stanzlinge (60 mm) der mit Initiator versehenen Membran im Stickstoffstrom entgast. Mittels einer Überführungskanüle wurde die Polymerisationslösung unter Schutzgas zu den Stanzlingen gegeben und es wurde unter Sauerstoffausschluss für 8 h gerührt. Die Polymerisation wurde durch Öffnung des Reaktionsgefäßes und durch Einleiten von Sauerstoff terminiert. Die mit Glycidylmethacrylat modifizierten Stanzlinge wurden mit Wasser und Aceton gewaschen, um Katalysatorreste und Polymer, das nicht auf der Oberfläche gebunden ist, zu entfernen. Das Zielprodukt wurde nach Trocknen für 6 h bei 40 °C erhalten. Der Pfropfgrad wurde gravimetrisch nach Gleichung (1) bestimmt.

| Pfropfgrad |
|---|
| 10 ± 1 % |

*Sulfonierung der Epoxidgruppen:*

Die Umsetzung zu einem starken Kationentauscher erfolgte folgendermaßen:
600 g Wasser wurden vorgelegt und es wurden unter Kühlung 180 g $Na_2SO_3$, 20 g $Na_2HPO_4 \cdot 2\ H_2O$ und 50 g TBABS gelöst. Der pH-Wert der Lösung wurde mit NaOH (32 %) auf 8 eingestellt und schrittweise Wasser zugegeben, bis 1 kg Lösung erreicht waren. Dabei wurde der pH-Wert regelmäßig kontrolliert und bei Abweichung mit NaOH (32 %) auf pH 8 nachreguliert.

Zur Umsetzung wurde so viel Lösung verwendet, dass alle Membranen frei in einer 500 ml Weithalsgewindeflasche schwimmen konnten. Die Lösung wurde auf 85 °C erwärmt und die Membranen unter Rühren 45 min bei 85 °C umgesetzt.

| Ionische Kapazität |
|---|
| 4,00 μmol/$cm^2$ |

<u>Vergleichsbeispiel 2: ATRP ohne Abstandshalter auf Fasern</u>

*Oberflächenaktivierung ohne Abstandshalter:*

In einer typischen Umsetzung wurden 5 g Fasern (Winged Fasern (PBT) oder 4DG™ Fasern (PET)) in einer Lösung aus 50 mL Ethylendiamin und 50 mL Ethanol bei 50 °C für 2 h gerührt. Nach Erreichen der gewünschten Umsatzdauer wurden die Fasern abfiltriert, in einen großen Überschuss an RO-Wasser gegeben und für 30 min gerührt. Im Anschluss wurde erneut abfiltriert und solange mit RO-Wasser gewaschen, bis das Filtrat keine basische Reaktion mehr zeigt. Die erhaltenen aminierten Fasern wurden nach einer Trocknung für 6 h bei 60 °C bezüglich Massenverlust und Amingruppendichte charakterisiert.

| Fasertyp | Amingruppendichte |
|---|---|
| Winged Fibers | 4,5 $nm^{-2}$ |

*Initiatorimmobilisierung :*

In einer typischen Immobilisierung wurden α-Bromisobutyrylbromid (1,00 mL, 1,86 g, 8,09 mmol) und Triethylamin (1,50 mL, 1,08 g, 10,7 mmol) zu 100 mL wasserfreiem Dichlormethan gegeben. In dieser Lösung wurden 4 g aminierte Fasern suspendiert und 2 h bei Raumtemperatur geschüttelt. Zur Aufarbeitung wurden langsam 10 mL Isopropanol tropfenweise zugegeben und weitere 30 min geschüttelt. Im Anschluss wurden die Fasern abfiltriert und mit

Wasser, Isopropanol, Aceton und wieder Wasser gewaschen. Nach einer Trocknung bei 40 °C über 12 h wurden die mit Initiator versehenen Fasern bezüglich der Initiatordichte charakterisiert.

| Fasertyp | Initiatordichte |
|---|---|
| Winged Fibers | 3,6 $nm^{-2}$ |

*Oberflächengebundene Polymerisation:*

In einer repräsentativen Polymerisation wurde Glycidylmethacrylat (2,5 g, 17,5 mmol, 175 Äq.) in 100 mL RO-Wasser und 100 mL Isopropanol gelöst. Die Lösung wurde im Anschluss für 30 min durch Einleiten von Stickstoff entgast. Im Stickstoffgegenstrom wurden Kupfer(I)bromid (15,0 mg, 104 μmol, 1,0 Äq.), 2,2'-Bipyridin (50,0 mg, 320 μmol, 3,1 Äq.) und Ascorbinsäure (45,0 mg, 225 μmol, 1,25 Äq.) hinzugegeben und es wurde für weitere 30 min unter Rühren entgast. Parallel wurden 1,0 g mit Initiator versehene Fasern im Stickstoffstrom entgast. Mittels einer Überführungskanüle wurde die Polymerisationslösung unter Schutzgas zu den Fasern gegeben und es wurde unter Sauerstoffausschluss für 8 h gerührt. Die Polymerisation wurde durch Öffnung des Reaktionsgefäßes und durch Einleiten von Sauerstoff terminiert. Die mit Glycidylmethacrylat modifizierten Fasern wurden mit Wasser und Aceton gewaschen um Katalysatorreste und Polymer, das nicht auf der Oberfläche gebunden ist, zu entfernen. Das Zielprodukt wurde nach Trocknen für 6 h bei 40 °C erhalten. Der Pfropfgrad wurde gravimetrisch nach Gleichung (1) bestimmt.

| Fasertyp | Pfropfgrad |
|---|---|
| Winged Fibers | 2 ± 0,5 % |

*Sulfonierung der Epoxidgruppen:*

Die Umsetzung zu einem starken Kationentauscher erfolgte folgendermaßen:
600 g Wasser wurden vorgelegt und es wurden unter Kühlung 180 g $Na_2SO_3$, 20 g $Na_2HPO_4 \cdot 2\,H_2O$ und 50 g TBABS gelöst. Der pH-Wert der Lösung wurde mit NaOH erreicht waren. Dabei wurde der pH-Wert regelmäßig kontrolliert und bei Abweichung mit NaOH (32 %) auf pH 8 nachreguliert.

Zur Umsetzung wurde so viel Lösung verwendet, dass alle Fasern frei in einer 500 ml Weithalsgewindeflasche schwimmen konnten. Die Lösung wurde auf 85 °C erwärmt und die Fasern unter Rühren 45 min bei 85 °C umgesetzt.

| Fasertyp | Ionische Kapazität |
|---|---|
| Winged Fibers | 100 ± 10 μmol/g |

Vergleichsbeispiel 3: ATRP ohne Abstandshalter auf Nvlonmembran

*Oberflächenaktivierung ohne Abstandshalter:*

In einer typischen Umsetzung wurden Stanzlinge (47 mm Durchmesser) einer Nylonmembran (Typ 21303) für 10 min in einer Lösung bestehend aus 0,5 wt.% Chloramin-T, 95,5 wt.% RO-Wasser und 4 wt.% Natrium-Acetatpuffer (1 M, pH = 6) bei Raumtemperatur geschüttelt. Nach der Umsetzung wurden die Membranen für 10 min in RO-Wasser gewaschen und 1 h bei 60 °C getrocknet.

*Oberflächengebundene Polymerisation:*

In einer repräsentativen Polymerisation wurde Glycidylmethacrylat (2,5 g, 17,5 mmol, 175 Äq.) in 100 mL RO-Wasser und 100 mL Isopropanol gelöst. Die Lösung wurde im Anschluss für 30 min durch Einleiten von Stickstoff entgast. Im Stickstoffgegenstrom wurden Kupfer(I)bromid (15,0 mg, 104 μmol, 1,0 Äq.), 2,2'-Bipyridin (50,0 mg, 320 μmol, 3,1 Äq.) und Ascorbinsäure (45,0 mg, 225 μmol, 1,25 Äq.) hinzugegeben und es wurde für weitere 30 min unter Rühren entgast. Parallel wurden 3 Stanzlinge (47 mm Durchmesser) im Stickstoffstrom entgast. Mittels einer Überführungskanüle wurde die Polymerisationslösung unter Schutzgas zu den Stanzlingen gegeben und es wurde unter Sauerstoffausschluss für 90 min gerührt. Die Polymerisation wurde durch Öffnung des Reaktionsgefäßes und durch Einleiten von Sauerstoff terminiert. Die mit Glycidylmethacrylat modifizierten Stanzlinge wurden mit Wasser und Aceton gewaschen um Katalysatorreste und Polymer, das nicht auf der Oberfläche gebunden ist, zu entfernen. Das Zielprodukt wurde nach Trocknen für 6 h bei 40 °C erhalten. Der Pfropfgrad wurde gravimetrisch nach Gleichung (1) bestimmt.

| Fasertyp | Pfropfgrad |
|---|---|
| Nylonmembran | 9 % |

*Sulfonierung der Epoxidgruppen:*

Die Umsetzung zu einem starken Kationentauscher erfolgte folgendermaßen: 600 g Wasser wurden vorgelegt und es wurden unter Kühlung 180 g $Na_2SO_3$, 20 g $Na_2HPO_4 \cdot 2\ H_2O$ und 50 g TBABS gelöst. Der pH-Wert der Lösung wurde mit NaOH (32 %) auf 8 eingestellt und es wurde schrittweise Wasser zugegeben, bis 1 kg Lösung erreicht waren. Dabei wurde der pH-Wert regelmäßig kontrolliert und bei Abweichung mit NaOH (32 %) auf pH 8 nachreguliert.

Zur Umsetzung wurde so viel Lösung verwendet, dass alle Stanzlinge frei in einer 500 ml Weithalsgewindeflasche schwimmen konnten. Die Lösung wurde auf 85 °C erwärmt und die Stanzlinge wurden unter Rühren 45 min bei 85 °C umgesetzt.

| Trägermaterial | Ionische Kapazität |
|---|---|
| Nylonmembran | 2,5 ± 0,2 $\mu$mol/cm$^2$ |

Vergleich zwischen erfindunqsqemäßen und herkömmlichen Adsorptionsmedien:

Die vorstehend synthetisierten erfindungsgemäßen Adsorptionsmedien wurden mit kommerziell erhältlichen Adsorptionsmedien und den vorstehend synthetisierten (nicht erfindungsgemäßen) Vergleichs-Adsorptionsmedien bezüglich des Verhältnisses zwischen statischer Proteinbindung zu ionischer Kapazität für Lyosym und IgG verglichen.

Die erfindungsgemäßen Adsorptionsmedien auf Basis von PBT zeigen durch die Anwesenheit eines flexiblen Abstandshalterelements 1,5- bis 3,6-fach erhöhte Werte für das Verhältnis zwischen statischer Proteinbindungskapazität und ionischer Bindungskapazität für Lysozym im Vergleich zu Chromatographiematrizen ohne dieses Abstandshalterelement. Für Immunoglobulin (IgG) ist das vorgenannte Verhältnis für die erfindungsgemäßen Adsorptionsmedien um den Faktor 1,7 bis 3,7 erhöht (siehe Tabelle 3 für erfindungsgemäße Adsorptionsmedien umfassend PBT).

Ein ähnliches Ergebnis ergibt sich für erfindungsgemäße Adsorptionsmedien auf Basis von PET: Durch die Anwesenheit eines flexiblen Abstandshalterelements resultieren für das Verhältnis zwischen statischer Proteinbindungskapazität und ionischer Bindungskapazität für Lysozym 1,2 bis 2,8-fach erhöhte Werte im Vergleich zu Chromatographiematrizen ohne dieses Abstandshalterelement. Für Immunoglobulin (IgG) ist das vorgenannte Verhältnis für die erfindungsgemäßen Adsorptionsmedien um den Faktor 1,4 bis 3 erhöht (siehe Tabelle 4 für erfindungsgemäße Adsorptionsmedien umfassend PET).

Tabelle 5 belegt ebenfalls, dass Chromatographiemedien mit Abstandshalterelementen für die Ausführungsform einer Nylonmembran ein 1,5 bis 4,5-fach erhöhtes Verhältnis zwischen statischer Proteinbindungskapazität und ionischer Kapazität aufweisen im Vergleich zu Medien ohne Abstandshalterelement, wobei die Erhöhung dieses Verhältnisses für das Splitfaservlies in einem vergleichbaren Bereich einer 1,5 bis 4-fachen Erhöhung liegt.

Diese Befunde belegen, dass bei den erfindungsgemäßen Adsorptionsmedien bereits bei einer geringen Ligandendichte hohe Bindungskapazitäten für Proteine erreicht werden können.

Tabelle 3:

| **Vergleichsparameter** | **Sartobind S[5]** | **Fractogel EMD[6] $SO_3^-$ (M)[1]** | **ATRP ohne Abstandshalter Winged Fasern (PBT)** | **ATRP mit PAA-Abstandshalter Winged Fasern (PBT)** |
|---|---|---|---|---|

(fortgesetzt)

| Vergleichsparameter | Sartobind S[5] | Fractogel EMD[6] $SO_3^-$ (M)[1] | ATRP ohne Abstandshalter Winged Fasern (PBT) | ATRP mit PAA-Abstandshalter Winged Fasern (PBT) |
|---|---|---|---|---|
| **Verhältnis statische Proteinbindung/ionische Kapazität für Lysozym und IgG [mg/μmol]** | 0,33 ± 0,05 Lysozym[2] 0,30 ± 0,02 IgG[3] | 0,77 ± 0,1 Lysozym[2] 0,66 ± 0,08 IgG[4] | 0,71 ± 0,12 Lysozym[2] 0,62 ± 0,1 IgG[3] | 1,19 ± 0,06 Lysozym[2] 1,11 ± 0,07 IgG[3] |

PBT: Polybutylenterephthalat; PAA: Polyallylamin
[1]E. Grushka, N. Grinberg, Advances in Chromatography, Vol. 47, 2009 und EP 2 153 877 A1
[2]KPi-Puffer 10 mM, pH = 7, LF = 1,75 mS/cm
[3]Natrium-Acetat Puffer 10 mM, pH = 5, 100 mM NaCl
[4]Natrium-Acetat Puffer 10 mM, pH = 5
[5]Vliesverstärkte regenerierte vernetzte Cellulosemembran mit Sulfonsäureliganden
[6]bestehend aus vernetzten Methacrylaten; Firma Merck

Tabelle 4:

| Vergleichsparameter | Sartobind S[5] | Fractogel EMD[6] $SO_3^-$ (M)[1] | ATRP ohne Abstandshalter 4DG™ Fasern (PET) | ATRP mit PAA-Abstandshalter 4DG™ Fasern (PET) |
|---|---|---|---|---|
| **Verhältnis statische Proteinbindung/ionische Kapazität für Lysozym und IgG [mg/μmol]** | 0,33 ± 0,05 Lysozym[2] 0,30 ± 0,02 IgG[3] | 0,77 ± 0,1 Lysozym[2] 0,66 ± 0,08 IgG[4] | 0,61 Lysozym[2] 0,57 IgG[3] | 0,91 ± 0,12 Lysozym[2] 0,90 ± 0,08 IgG[3] |

PET: Polyethylenterephthalat; PAA: Polyallylamin
[1]E. Grushka, N. Grinberg, Advances in Chromatography, Vol. 47, 2009 und EP 2 153 877 A1
[2]KPi-Puffer 10 mM, pH = 7, LF = 1,75 mS/cm
[3]Natrium-Acetat Puffer 10 mM, pH = 5, 100 mM NaCl
[4]Natrium-Acetat Puffer 10 mM, pH = 5
[5]Vliesverstärkte regenerierte vernetzte Cellulosemembran mit Sulfonsäureliganden
[6]bestehend aus vernetzten Methacrylaten; Firma Merck

Tabelle 5:

| Vergleichsparameter | Sartobind S[5] | Fractogel EMD[6] $SO_3^-$(M)[1] | ATRP ohne Abstandshalter Nylonmembran | ATRP mit PAA-Abstandshalter Nylonmembran | ATRP mit PAA-Abstandshalter auf Splitfaservlies |
|---|---|---|---|---|---|

(fortgesetzt)

| Vergleichsparameter | Sartobind S[5] | Fractogel EMD[6] $SO_3^-$(M)[1] | ATRP ohne Abstandshalter Nylonmembran | ATRP mit PAA-Abstandshalter Nylonmembran | ATRP mit PAA-Abstandshalter auf Splitfaservlies |
|---|---|---|---|---|---|
| **Verhältnis statische Proteinbindung/ ionische Kapazität für Lysozym und IgG [mg/ pmol]** | 0,33 ± 0,05 Lysozym[2] 0,30 ± 0,02 IgG[3] | 0,77 ± 0,1 Lysozym[2] 0,66 ± 0,08 IgG[3] | 0,30 ± 0,12 Lysozym[2] 0,22 ± 0,05 IgG[4] | 1,12 ± 0,08 Lysozym[2] 0,99 ± 0,09 IgG[3] | 1,19 ± 0,09 Lysozym[2] 1,01 ± 0,09 IgG[3] |

PAA: Polyallylamin
[1]E. Grushka, N. Grinberg, Advances in Chromatography, Vol. 47, 2009 und EP 2 153 877 A1
[2]KPi-Puffer 10 mM, pH = 7, LF = 1,75 mS/cm
[3]Natrium-Acetat Puffer 10 mM, pH = 5, 100 mM NaCl
[4]Natrium-Acetat Puffer 10 mM, pH = 5
[5]Vliesverstärkte regenerierte vernetzte Cellulosemembran mit Sulfonsäureliganden
[6]bestehend aus vernetzten Methacrylaten; Firma Merck

Abhängigkeit der Permeabilität von der Struktur der Polymerketten mit Liganden

Aufgabe der vorliegenden Erfindung ist es unter anderem, ein Adsorptionsmedium bereitzustellen, in welchem die chromatographisch aktiven Zentren (Liganden) optimal als Bindungsstellen ausgenutzt werden können, so dass die Anzahl der Liganden im Adsorbtionsmedium reduziert werden kann. Dies wird erfindungsgemäß dadurch gelöst, dass die Polymerketten mit Liganden aufgrund ihrer geringen Vernetzung und aufgrund der flexiblen polymeren Abstandshalterelemente eine hohe Flexibilität aufweisen. Daraus resultiert ein großer Wert für das Verhältnis von statischer Proteinbindungskapazität und ionischer Kapazität, wie vorstehend gezeigt wurde. Auf diese Weise kann die Zahl ionischer Liganden sehr weit reduziert werden. Daraus folgt:

- Geringer Bedarf an Ligand in der Herstellung;
- Optimierung der Permeabilität durch geringere Quellung des Pfropfpolymers.

Um den Einfluss der Polymerketten mit Liganden auf die Permeabilität abzubilden, wurden Membranen aus regenerierter, vernetzter Cellulose mittels ATRP ohne Abstandshalter modifiziert und zu starken Anionentauschern umgesetzt und dabei Kettendichte, Kettenlänge und Ligandendichte variiert.

*Kettenlänge:*

Mittels unterschiedlicher Polymerisationszeiten wurde die Kettenlänge bei identischer Kettendichte variiert. In Abhängigkeit der Kettenlänge wurde die Permeabilität des Adsorptionsmediums ermittelt (siehe Figur 3).
Wie aus Figur 3 ersichtlich, sinkt mit steigender Kettenlänge und somit höherer Ligandenzahl die Permeabilität. Vorteilhafterweise kann somit durch die vorliegende Erfindung, in welcher aufgrund der optimalen Ausnutzung der Liganden als Bindungsstellen ein geringerer Bedarf an Liganden besteht, eine Verminderung der Permeabilität des Adsorptionsmediums vermieden werden.

*Kettendichte:*

Mittels unterschiedlicher Dichte des oberflächengebundenen ATRP-Initiators konnte die Kettendichte variiert werden. In Abhängigkeit der ermittelten Initiatordichte wurde bei gleicher Polymerkettenlänge die Permeabilität der Membran ermittelt (siehe Figur 4).
Wie aus Figur 4 ersichtlich, sinkt mit steigender Kettendichte und somit höherer Ligandenzahl die Permeabilität. Vorteilhafterweise kann somit durch die vorliegende Erfindung, in welcher aufgrund der optimalen Ausnutzung der Liganden als Bindungsstellen ein geringerer Bedarf an Polymerketten mit Liganden besteht, eine Verminderungen der Permeabilität des Adsorptionsmediums vermieden werden.

*Ligandendichte:*

Durch Copolymerisation von 2-(Methacryloyloxy)ethyltrimethylammoniumchlorid mit nicht-ionischen bzw. inerten Monomeren, wie Hydroxyethylmethacrylat (HEMA), wurde die Ligandendichte im Hydrogel bei gleicher Polymerkettenlänge und -dichte variiert. In Abhängigkeit der erhaltenen ionischen Kapazität wurde die Permeabilität der Membran ermittelt (siehe Figur 5).

Wie aus Figur 5 ersichtlich, führt eine Reduktion der ionischen Funktionalitäten bei identischer Kettenlänge und -dichte zu einer Erhöhung der Permeabilität.

Zusammengefasst lässt sich daher feststellen, dass generell eine Erhöhung der ionischen Funktionalitäten innerhalb der Polymerketten, bedingt durch höhere Kettenlänge und -dichte oder Ligandendichte entlang der Polymerketten, zu einer Abnahme der Permeabilität führen. Die vorliegende Erfindung stellt daher vorteilhafterweise ein Adsorptionsmedium bereit, welches eine optimale Ausnutzung der Ligandenstellen ermöglicht und bei möglichst niedriger Ligandendichte hohe Bindungskapazitäten für Proteine erzielt.

Einfluss der Kettendichte auf die Zugänglichkeit der Polymerketten mit Liganden:

Wie bereits dargestellt, zeigen modifizierte Winged Fasern ein bedeutend höheres Verhältnis aus statischer Proteinbindungskapazität und ionischer Kapazität als Membranen mit einer vergleichbaren Initiatordichte. Im untersuchten Bereich scheint die Kettendichte keinen signifikanten Einfluss auf das zur Verfügung stehende Bindungsvolumen zu haben. Weitere Untersuchungen für deutlich längere Aminolysezeiten und daraus folgend höhere Amingruppen- bzw. Initiator/Kettendichten zeigen jedoch einen dem Membranmodell ähnlichen Verlauf. Es ist zu beobachten, dass auch bei sehr hohen erzielten Kettendichten bedeutend mehr freies Bindungsvolumen zur Verfügung steht. Bei kurzen Aminolysedauern und somit geringeren Kettendichten kann ein Plateau beobachtet werden. Das Verhältnis aus statischer Proteinbindung und ionischer Kapazität nimmt einen nahezu konstanten Wert an (siehe Figur 6).

Somit lässt sich ein optimaler Bereich von 0,1 bis 30 $nm^{-2}$ für Amingruppen- bzw Initiatordichten bestimmen.

Vernetzung der Polymerschicht mit Liganden eines starken Anionentauschers durch bifunktionelle Monomere:

Eine Vernetzung der Polymerketten mit Liganden kann im Allgemeinen durch eine Copolymerisation von monofunktionellen und bifunktionellen Monomeren gesteuert werden. Der Anteil des Vernetzers (bifunktionelles Monomer) erlaubt eine direkte Steuerung des Vernetzungsgrades und bei Kenntnis der Copolymerisationsparameter auch eine Quantifizierung.

Zur Herstellung von starken Anionentauschermaterialien wurde eine SI-ATRP ("*surface initiated atom transfer radical polymerization*"; oberflächengebundene Atomtransferradikalpolymerisation) der Monomere 2-(Methacryloyloxy)ethyl-trimethylammoniumchlorid (METAC) und Methylenbisacrylamid auf unterschiedlichen Trägermaterialien durchgeführt. Das Verhältnis aus Proteinbindungskapazität und ionischer Kapazität wurde nach bekannten Methoden durchgeführt. Die Ergebnisse für eine Polymerisation ohne Vernetzer innerhalb der Hydrogelschicht sind in Figur 7 dargestellt. Als Vergleichswert ist der Wert für Sartobind® Q als Beispiel einer unkontrollierten radikalischen Polymerisation mit vergleichbarer Ligandenchemie mit aufgeführt. Die Bindung wurde gegenüber BSA in TRIS/HCl-Puffer (pH=7.2, LF=1.8 mS/cm) als Modellprotein ermittelt.

Auf beiden Modellsystemen ("Winged Fasern" mit Abstandshalter und Cellulosemembran ohne Abstandshalter) wurde eine kontrollierte Vernetzung der Polymerketten mit Liganden durch Zusatz verschiedener Anteile an Methylenbisacrylamid erreicht. Der Einfluss auf das Verhältnis der statischen Proteinkapazität und ionischen Kapazität ist in den Figuren 8 und 9 dargestellt.

Für beide Trägermedien ist eine deutliche Abnahme der Ligandenzugänglichkeit zu beobachten. Diese fällt für eine Polymerisation mit Abstandshalter deutlich stärker aus. Durch diese Beobachtungen zeigt sich, dass der Vernetzungsgrad direkt mit der Ligandenzugänglichkeit zusammenhängt und dass für die Polymerketten mit Liganden ein Grenzwert möglichst nicht überschritten werden sollte.

Die Figuren zeigen:

Figur 1: Schematischer Durchbruch einer Titration mit 10 mM HCl-Lösung.

Figur 2: Kalibriergerade zur Bestimmung der Menge an Bromid in einer Probe.

Figur 3: Permeabilität in Abhängigkeit der Kettenlänge.

Figur 4: Permeabilität in Abhängigkeit der Initiatordichte.

Figur 5: Permeabilität in Abhängigkeit der Ligandendichte.

Figur 6: Abhängigkeit des Verhältnisses aus statischer Lysozymbindungskapazität und ionischer Kapazität von der Amingruppendichte.

Figur 7: Verhältnis aus statischer Proteinbindungskapazität zu ionischer Kapazität.

Figur 8: Verhältnis aus statischer Proteinbindungskapazität zu ionischer Kapazität auf einer regenerierten, vernetzten Cellulosemembran.

Figur 9: Verhältnis aus statischer Proteinbindungskapazität zu ionischer Kapazität auf "PBT Winged Fasern" mit einem Polyallylamin-Abstandshalter.

## Patentansprüche

1. Adsorptionsmedium, insbesondere Chromatographiemedium, umfassend eine Chromatographiematrix; polymere Abstandshalterelemente, die an die Oberfläche der Chromatographiematrix gebunden sind; und Polymerketten mit chromatographisch aktiven Zentren, wobei die Polymerketten an die polymeren Abstandshalterelemente gebunden sind und wobei die Polymerketten Polyacrylate oder Polymethacrylate sind.

2. Adsorptionsmedium nach Anspruch 1, wobei die Chromatographiematrix ein Material umfasst, ausgewählt aus der Gruppe, bestehend aus natürlichen oder synthetischen Fasern, Polymer-Membranen, Polymergelen, Folien, Vliesen und Geweben.

3. Adsorptionsmedium nach Anspruch 1 oder 2, wobei die polymeren Abstandshalterelemente ausgewählt sind aus der Gruppe, bestehend aus Polyaminen, Polyalkoholen, Polythiolen, Polyacrylaten, Polymethacrylaten, Poly(meth)acrylamiden, Poly-N-alkyl(meth)acrylamiden sowie Copolymeren, bestehend aus zwei oder mehr der vorstehenden Polymere, und Copolymeren, bestehend aus einem oder mehreren der vorstehenden Polymere und aus Polymeren, die keine nucleophilen funktionellen Gruppen tragen.

4. Adsorptionsmedium nach Anspruch 3, wobei die polymeren Abstandshalterelemente Polyamine sind.

5. Adsorptionsmedium nach einem der Ansprüche 1 bis 4, wobei die polymeren Abstandshalterelemente keine Epoxygruppen aufweisen.

6. Adsorptionsmedium nach einem der Ansprüche 1 bis 5, wobei das Adsorptionsmedium einen Pfropfgrad von 1% bis 50% aufweist, wobei der Propfgrad durch die folgende Gleichung beschrieben wird: Pfropfgrad [%] = (Gewicht des Adsorptionsmediums mit Polymerketten - Gewicht des Adsorptionsmediums ohne Polymerketten) / (Gewicht des Adsorptionsmediums ohne Polymerketten) * 100%.

7. Adsorptionsmedium nach einem der Ansprüche 1 bis 6, wobei die funktionelle Gruppendichte der Chromatographiematrix von 1,5 bis 30 $nm^{-2}$ beträgt.

8. Adsorptionsmedium nach einem der Ansprüche 1 bis 7, wobei die chromatographisch aktiven Zentren der Polymerketten ausgewählt sind aus der Gruppe, bestehend aus anionischen und kationischen Gruppen, hydrophoben Gruppen, Affinitätsliganden, Metallchelaten und reaktiven Epoxid-, Aldehyd-, Azlacton-, N-Hydroxysuccinimid- und/oder Carbodiimid-Gruppen.

9. Verfahren zur Herstellung eines Adsorptionsmediums nach einem der Ansprüche 1 bis 8, umfassend die Schritte:

   (a) Bereitstellen einer Chromatographiematrix;
   (b) Immobilisieren von polymeren Abstandshalterelementen auf der Oberfläche der Chromatographiematrix; und
   (c) Immobilisieren von Polymerketten mit chromatographisch aktiven Zentren auf den polymeren Abstandshalterelementen, wobei die Polymerketten Polyacrylate oder Polymethacrylate sind.

10. Verfahren zur Herstellung eines Adsorptionsmediums nach Anspruch 9, wobei der Schritt (c) die Schritte

(c1) Immobilisieren eines Polymerisationsinitiators auf den polymeren Abstandshalterelementen;
(c2) Durchführen einer Abstandshalterelement-gebundenen Polymerisation eines Monomers zum Bilden von immobilisierten Polymerketten auf den polymeren Abstandshalterelementen; und
(c3) Modifizieren der immobilisierten Polymerketten zum Bilden von chromatographisch aktiven Zentren auf den Polymerketten umfasst.

**11.** Verfahren zur Herstellung eines Adsorptionsmediums nach Anspruch 10, wobei die Abstandshalterelement-gebundene Polymerisation im Schritt (c2) mittels einer Atomtransferradikalpolymerisation (ATRP) durchgeführt wird.

**12.** Verfahren zur Herstellung eines Adsorptionsmediums nach Anspruch 10 oder 11, wobei im Schritt (c2) eine Polymerisationslösung verwendet wird, die einen Anteil von höchstens 3 mol-% an bifunktionellen Monomeren, bezogen auf die Gesamtmenge der Monomere in der Lösung, aufweist.

**13.** Verwendung des Adsorptionsmediums nach einem der Ansprüche 1 bis 8 zur Aufreinigung von Biomolekülen, wobei die Biomoleküle Proteine, Peptide, Aminosäuren, Nukleinsäuren, Viren, Virus-artige Partikel und/oder Endotoxine sind.

## Claims

**1.** An adsorption medium, especially a chromatography medium, comprising a chromatography matrix;
polymeric spacer elements which have been bonded to the surface of the chromatography matrix; and
polymer chains containing chromatographically active centers, wherein the polymer chains have been bonded to the polymeric spacer elements and wherein the polymer chains are polyacrylates or polymethacrylates.

**2.** The adsorption medium as claimed in claim 1, wherein the chromatography matrix comprises a material selected from the group consisting of natural or synthetic fibers, polymer membranes, polymer gels, films, nonwovens and wovens.

**3.** The adsorption medium as claimed in claim 1 or 2, wherein the polymeric spacer elements are selected from the group consisting of polyamines, polyalcohols, polythiols, polyacrylates, polymethacrylates, poly(meth)acrylamides, poly-N-alkyl(meth)acrylamides and also copolymers consisting of two or more of the above polymers, and copolymers consisting of one or more of the above polymers and of polymers which do not bear nucleophilic functional groups.

**4.** The adsorption medium as claimed in claim 3, wherein the polymeric spacer elements are polyamines.

**5.** The adsorption medium as claimed in any of claims 1 to 4, wherein the polymeric spacer elements do not have epoxy groups.

**6.** The adsorption medium as claimed in any of claims 1 to 5, wherein the adsorption medium has a degree of grafting of from 1% to 50%, wherein the degree of grafting is described by the following equation: Degree of grafting [%] = (Weight of the adsorption medium with polymer chains - Weight of the adsorption medium without polymer chains) / (Weight of the adsorption medium without polymer chains) * 100%.

**7.** The adsorption medium as claimed in any of claims 1 to 6, wherein the functional group density of the chromatography matrix is from 1.5 to 30 $nm^{-2}$.

**8.** The adsorption medium as claimed in any of claims 1 to 7, wherein the chromatographically active centers of the polymer chains are selected from the group consisting of anionic and cationic groups, hydrophobic groups, affinity ligands, metal chelates and reactive epoxide, aldehyde, azlactone, N-hydroxysuccinimide and/or carbodiimide groups.

**9.** A method for producing an adsorption medium as claimed in any of claims 1 to 8, comprising the steps:

(a) providing a chromatography matrix;
(b) immobilizing polymeric spacer elements on the surface of the chromatography matrix; and
(c) immobilizing polymer chains containing chromatographically active centers on the polymeric spacer elements, wherein the polymer chains are polyacrylates or polymethacrylates.

**10.** The method for producing an adsorption medium as claimed in claim 9, wherein step (c) comprises the steps

(c1) immobilizing a polymerization initiator on the polymeric spacer elements;
(c2) carrying out a spacer element-initiated polymerization of a monomer to form immobilized polymer chains on the polymeric spacer elements; and
(c3) modifying the immobilized polymer chains to form chromatographically active centers on the polymer chains.

**11.** The method for producing an adsorption medium as claimed in claim 10, wherein the spacer element-initiated polymerization in step (c2) is carried out by means of an atom transfer radical polymerization (ATRP).

**12.** The method for producing an adsorption medium as claimed in claim 10 or 11, wherein use is made in step (c2) of a polymerization solution comprising a proportion of not more than 3 mol% of bifunctional monomers, based on the total amount of monomers in the solution.

**13.** The use of the adsorption medium as claimed in any of claims 1 to 8 for the purification of biomolecules, wherein the biomolecules are proteins, peptides, amino acids, nucleic acids, viruses, virus-like particles and/or endotoxins.

## Revendications

**1.** Milieu d'adsorption, en particulier milieu de chromatographie, comprenant une matrice de chromatographie; des éléments polymères d'espacement, liés à la surface de la matrice de chromatographie; et des chaînes polymères avec des centres actifs sur le plan chromatographique, les chaînes polymères étant liées aux éléments polymères d'espacement et les chaînes polymères étant des polyacrylates ou des polyméthacrylates.

**2.** Milieu d'adsorption selon la revendication 1, la matrice de chromatographie comprenant une matière sélectionnée parmi le groupe consistant en fibres naturelles ou synthétiques, en membranes polymères, en gels polymères, en feuilles, en non-tissé et en tissus.

**3.** Milieu d'adsorption selon la revendication 1 ou 2, les éléments polymères d'espacement étant sélectionnés parmi le groupe consistant en polyamines, polyalcools, polythiols, polyacrylates, polyméthacrylates, poly(méth)acrylamides, poly-N-alkyl(méth)acrylamides ainsi qu'en copolymères consistant en deux des polymères susmentionnés, ou plus, et en copolymères consistant en un ou plusieurs des polymères susmentionnés, et en polymères ne portant pas de groupements fonctionnels nucléophiles.

**4.** Milieu d'adsorption selon la revendication 3, les éléments polymères d'espacement étant des polyamines.

**5.** Milieu d'adsorption selon l'une des revendications 1 à 4, les éléments polymères d'espacement ne comportant pas de groupements époxy.

**6.** Milieu d'adsorption selon l'une des revendications 1 à 5, le milieu d'adsorption présentant un degré de greffage allant de 1% à 50%, le degré de greffage étant décrit par l'équation suivante: degré de greffage [%] = (masse du milieu d'adsorption avec chaînes polymères - masse du milieu d'adsorption sans chaînes polymères)/(masse du milieu d'adsorption sans chaînes polymères) * 100%.

**7.** Milieu d'adsorption selon l'une des revendications 1 à 6, la densité fonctionnelle des groupements de la matrice de chromatographie allant de 1,5 à 30 $nm^{-2.}$

**8.** Milieu d'adsorption selon l'une des revendications 1 à 7, les centres actifs sur le plan chromatographique des chaînes polymères étant sélectionnés parmi le groupe consistant en groupements anioniques et cationiques, groupements hydrophobes, ligands par affinité, chélates de métaux et groupements réactifs époxyde, aldéhyde, azlactone, N-hydroxysuccinimide et/ou carbodiimide.

**9.** Procédé de fabrication d'un milieu d'adsorption selon l'une des revendications 1 à 8, comprenant les étapes suivantes:

(a) fourniture d'une matrice de chromatographie;
(b) immobilisation d'éléments polymères d'espacement à la surface de la matrice de chromatographie; et

(c) immobilisation des chaînes polymères avec des centres actifs sur le plan chromatographique sur les éléments polymères d'espacement, les chaînes polymères étant des polyacrylates ou des polyméthacrylates.

**10.** Procédé de fabrication d'un milieu d'adsorption selon la revendication 9, l'étape (c) comprenant les étapes suivantes:

(c1) immobilisation d'un initiateur de polymérisation sur les éléments polymères d'espacement;
(c2) réalisation d'une polymérisation liée par des éléments d'espacement, d'un monomère pour la formation de chaînes polymères immobilisées sur les éléments polymères d'espacement; et
(c3) modification des chaînes polymères immobilisées pour la formation de centres actifs sur le plan chromatographique sur les chaînes polymères.

**11.** Procédé de fabrication d'un milieu d'adsorption selon la revendication 10, la polymérisation liée par des éléments d'espacement à l'étape (c2) se fait au moyen d'une polymérisation radicalaire par transfert d'atome (ATRP).

**12.** Procédé de fabrication d'un milieu d'adsorption selon la revendication 10 ou 11, à l'étape (c2), une solution de polymérisation étant utilisée qui présente une fraction de tout au plus 3% en mole en monomères bifonctionnels, sur base de la quantité totale des monomères dans la solution.

**13.** Utilisation du milieu d'adsorption selon l'une des revendications 1 à 8 pour la purification de biomolécules, les biomolécules étant des protéines, des peptides, des acides aminés, des acides nucléiques, des virus, des particules de type virus et/ou des endotoxines.

Figur 1

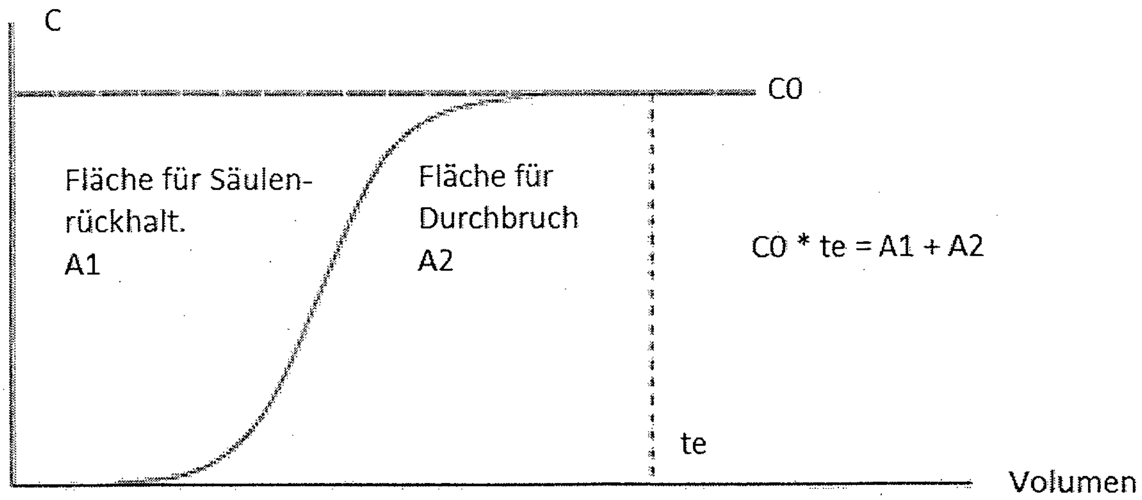


Figur 2

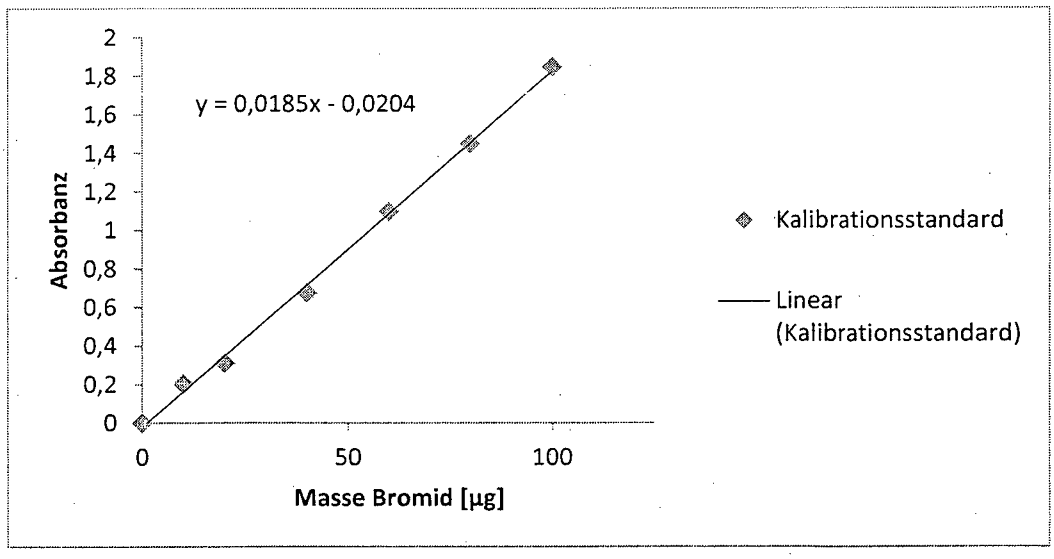

Figur 3

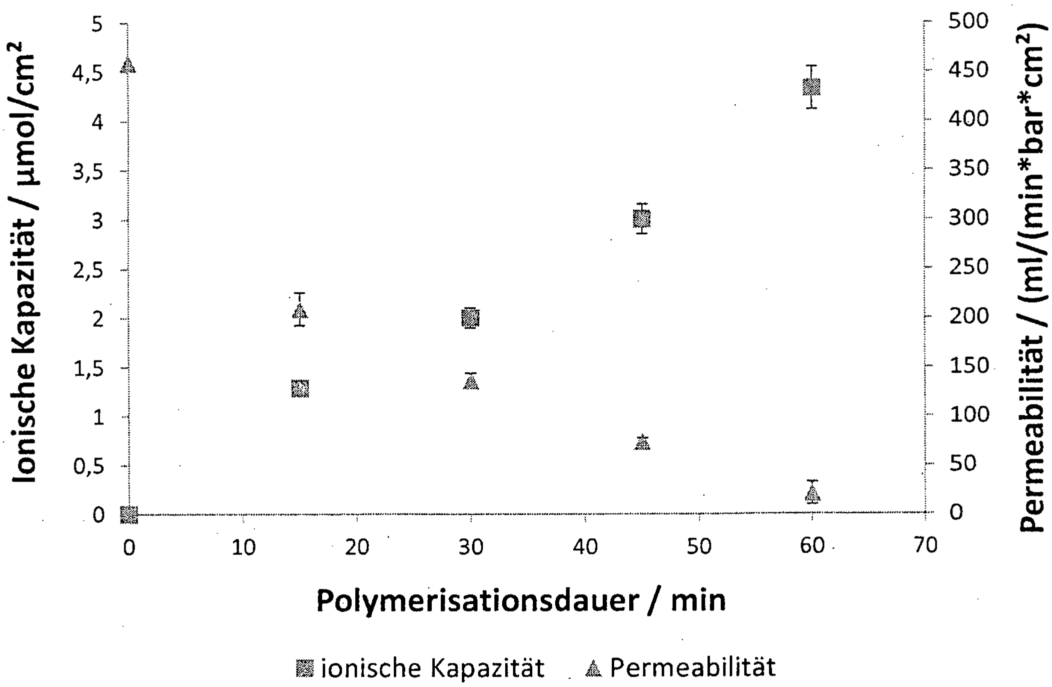


Figur 4

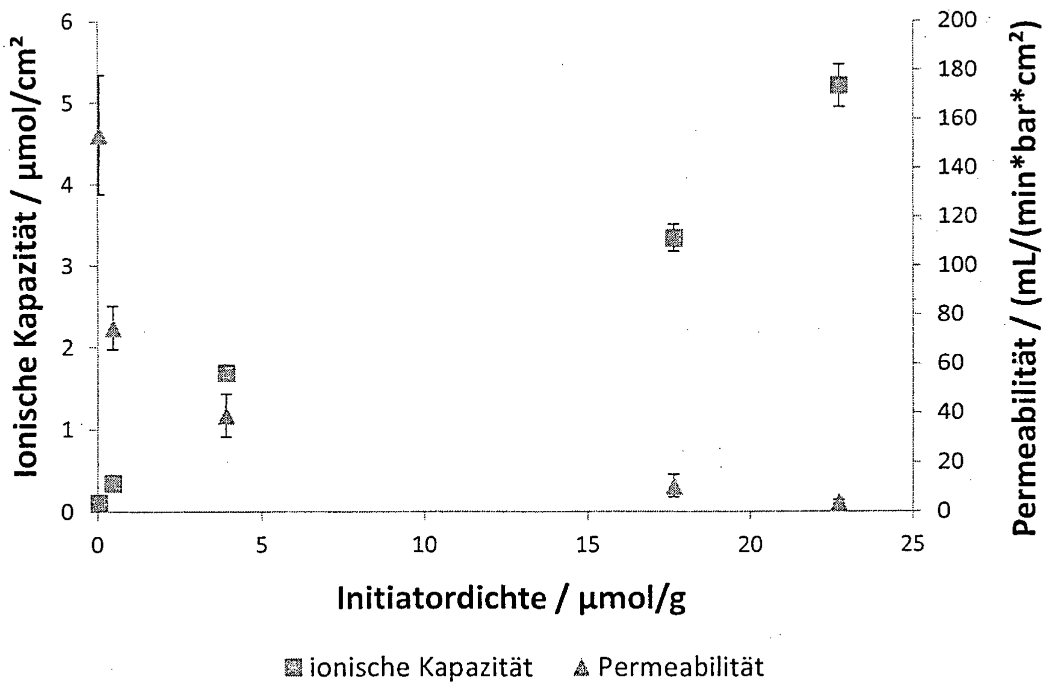

Figur 5

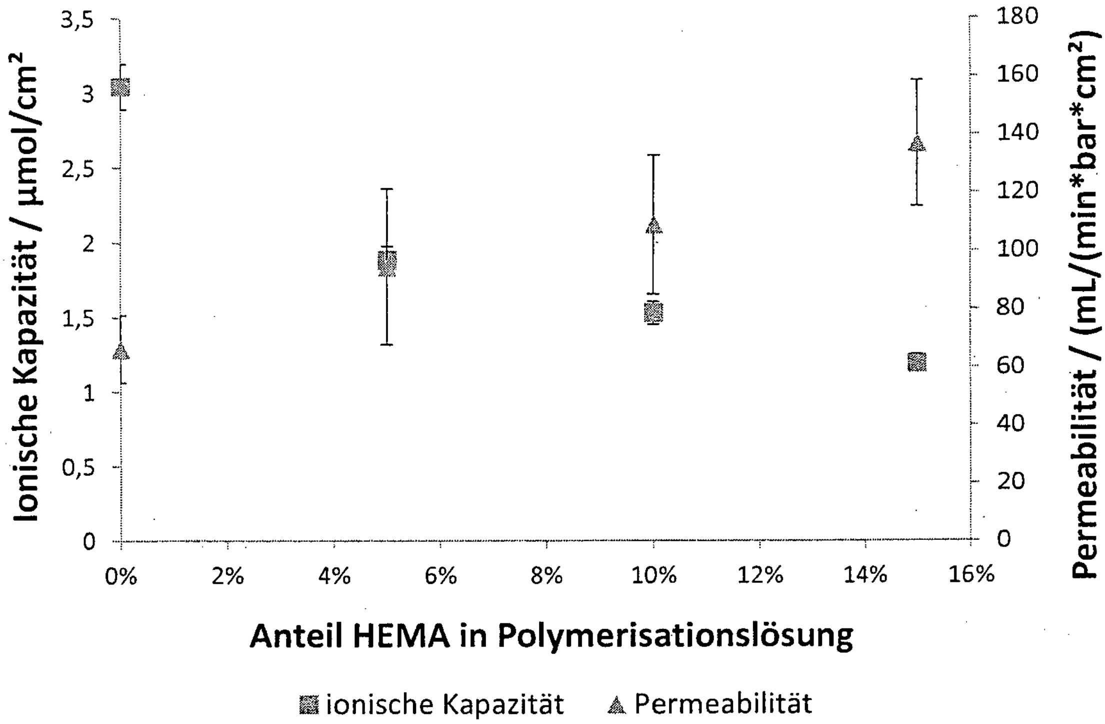


Figur 6

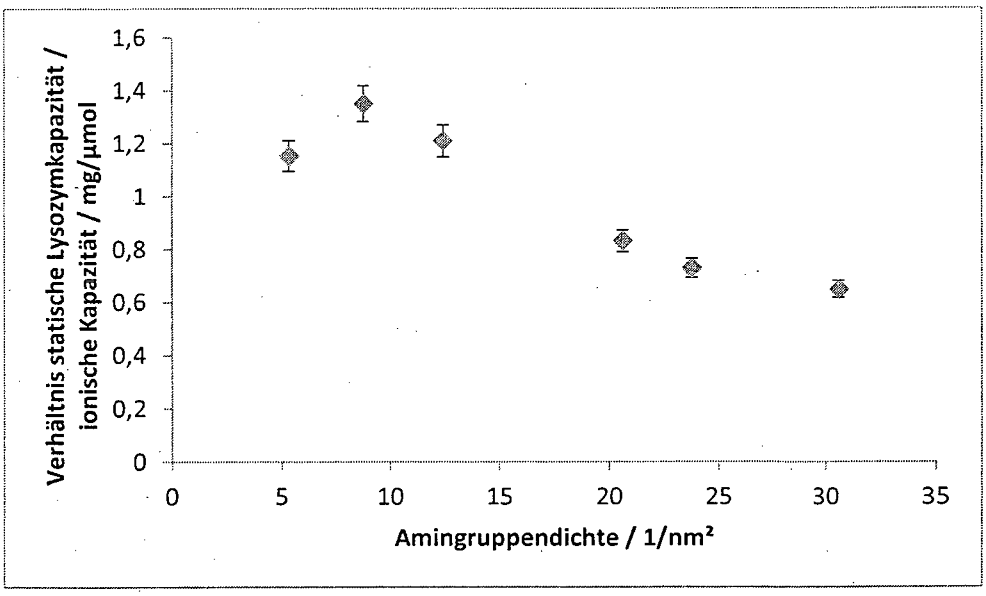

Figur 7

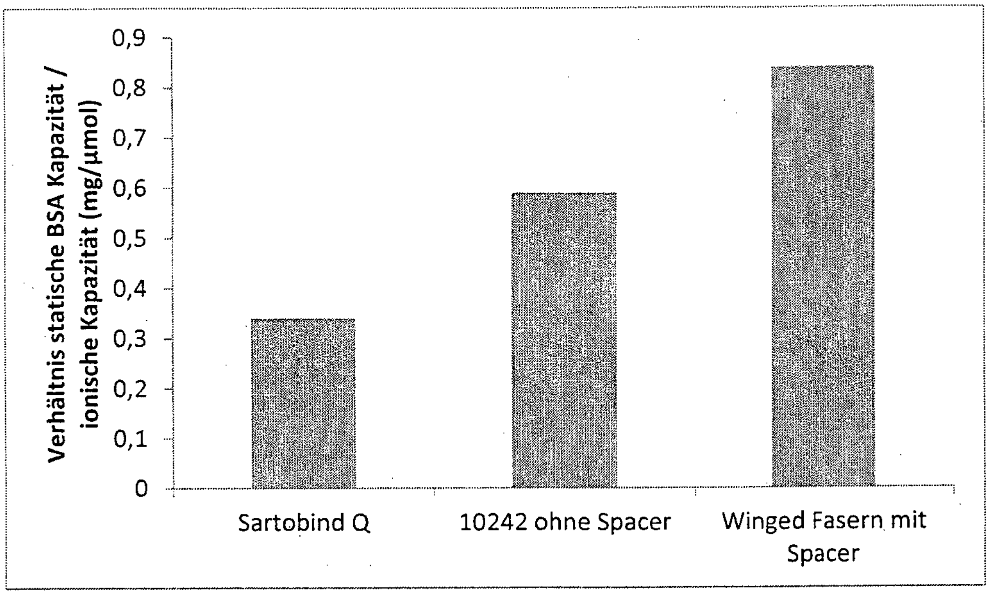


Figur 8

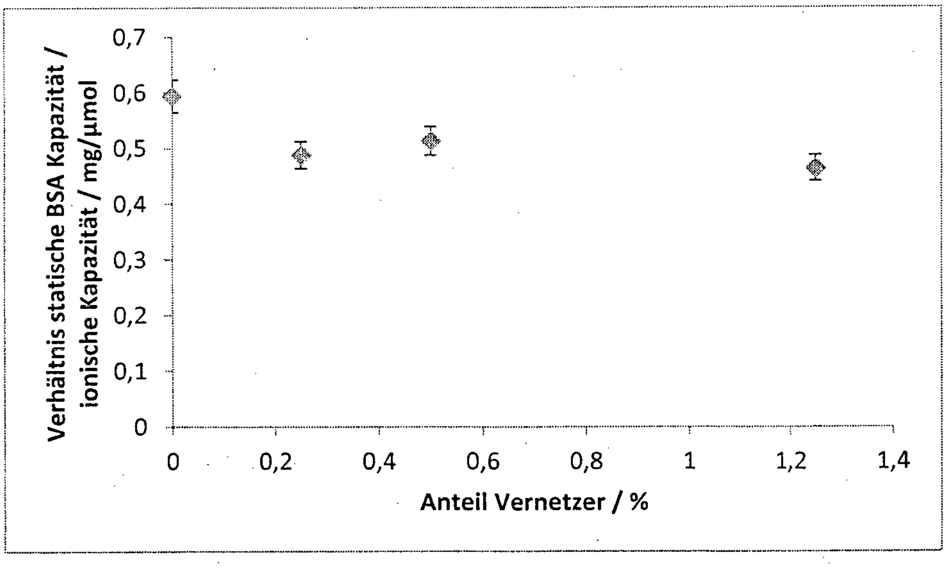

Figur 9

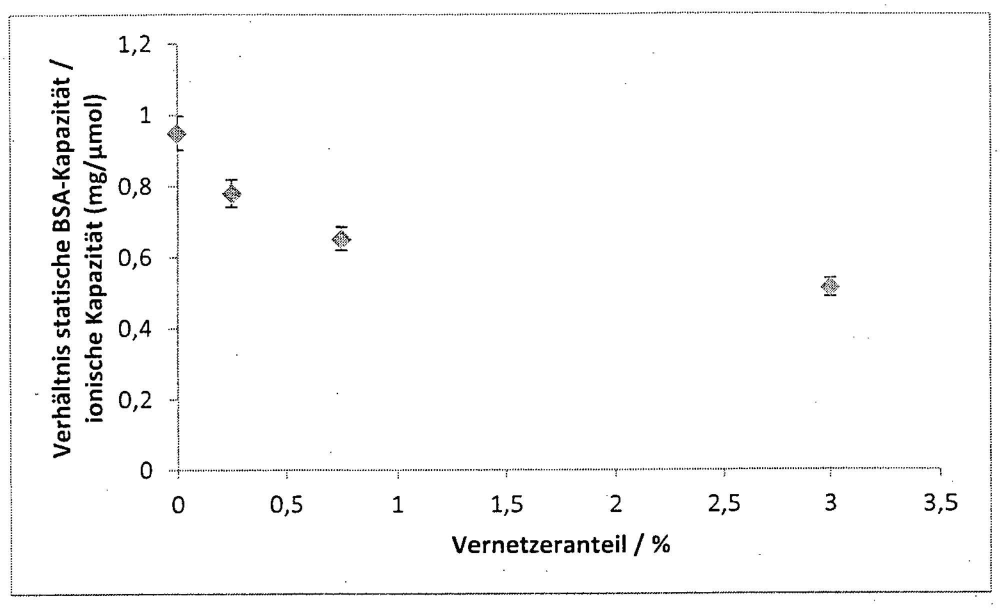
Verhältnis statische BSA-Kapazität / ionische Kapazität (mg/µmol)
1,2
1
0,8
0,6
0,4
0,2
0
0
0,5
1
1,5
2
2,5
3
3,5
Vernetzeranteil / %

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2014171437 A1
- WO 2014034644 A1
- WO 2014067605 A1
- EP 2153877 A1


### In der Beschreibung aufgeführte Nicht-Patentliteratur


- **GREG T. HERMANSON ; A. KRISHNA MALLIA ; PAUL K. SMITH.** Immobilized Affinity Ligand Techniques. Academic Press, INC, 1992
- **X. QIAN et al.** *Applied Surface Science,* 2013, vol. 271, 240-247
- **G. T. HERMANSON et al.** Immobilized Affinity Ligand Techniques. Academic Press, INC, 1992
- **NOEL et al.** *Bioconjugate Chemistry,* 2011, vol. 22, 1690-1699
- **E. GRUSHKA ; N. GRINBERG.** *Advances in Chromatography,* 2009, vol. 47